# EUROPEAN PATENT APPLICATION

(11) **EP 2 179 659 A1**
(43) Date of publication of application: **28.04.2010**
(21) Application number: 08791068.3
(22) Date of filing: 10.07.2008
(51) Int. Cl.: A23K 1/16, A23K 1/175, A23K 1/18, A23L 1/30

(54) **CALCIUM ABSORPTION ENHANCER**

(30) Priority: 13.07.2007 JP 2007184702
(71) Applicant: Menicon Co., Ltd., Nagoya-shi Aichi 460-0006 (JP); Kawai Hiryo Kabushikigaisha, Shizuoka 438-0068 (JP); Nagase Sanbio Co., Ltd., Tokyo 103-0024 (JP)
(72) Inventor: MATANO, Yutaka, Nagoya-shi Aichi 461-0003 (JP); MATSUI, Yuri, Nagoya-shi Aichi 461-0003 (JP); KAWAI, Hidemi, Iwata-shi Shizuoka 438-0068 (JP); SATO, Nobumitsu, Osaka-shi Osaka 550-0013 (JP)
(74) Representative: Sherrard-Smith, Hugh
(86) International application number: PCT/JP2008/062520
(87) International publication number: WO 2009/011288

(57) **Abstract**

The present invention provides a calcium absorption enhancer that places no burden on the body of a human or a domestic animal to which a calcium compound is administered and can increase the efficiency with which a calcium content is absorbed into the body by supplying calcium ions in the stomach or the like of the human or the domestic animal in a sustained-release manner after administration of the calcium compound. The calcium absorption enhancer of the present invention contains, as an active ingredient, water-soluble calcium particles that can release calcium ions in an aqueous solution in a sustained-release manner.

## Description

### Technical Field

The present invention relates to a calcium absorption enhancer. More specifically, the present invention relates to a calcium absorption enhancer that enables calcium, which constitutes the body of a human or a domestic animal and is an essential life-sustaining component, to be efficiently absorbed into the body.

### Background Art

Today, the incidence of various bone diseases, such as osteoporosis, fracture, and low back pain, tends to increase and these diseases have become problematic particularly in an aging society. Currently, research and development for prevention and treatment of various bone diseases are ongoing.

In the advancement of research and development for prevention and treatment of various bone diseases, studies about bones and calcium metabolism are rapidly making progress at present. Of bone diseases, for example, osteoporosis is thought to be caused by, for example, insufficient intake of a calcium content, a decreased calcium ion absorbing ability, and postmenopausal hormone imbalance. Therefore, enhancement of absorption of calcium ions appears to be one of effective measures for preventing or treating bone diseases.

Appropriate bone mass acquisition and increases in the calcium content in the body (hereinafter referred to as "calcium fortification") are important during the breeding stage of domestic animals, such as poultry, livestock, cultured fish, and land-dwelling pet animals, in addition to humans.

Among domestic animals, it is important for poultry, for example, chickens to maintain adequate bone strength according to the stage of growth. Since poultry farming is generally conducted in a poultry house with a limited space, chickens tend to have a small amount of exercise. Therefore, poultry bone may become even more fragile. If chickens do not maintain adequate bone strength, fracture easily occurs during breeding before they grow to an intended size. This may reduce the commercial value of chickens.

Further, there is a problem that poultry having an inadequate calcium content in the body cannot produce chicken eggs with an abundant calcium content. Among poultry, for example, chickens are known to ingest a large amount of fluid particularly during a high-temperature season. In a chicken which has ingested a large amount of fluid, the gastric juice in the gizzard is diluted, reducing the concentration of hydrochloric acid, which is the gastric acid. In such a case, even if a calcium content such as calcium carbonate is abundantly mixed in feed components for calcium fortification, solubility of the calcium content is reduced in the gizzard of the chicken, making it difficult to elute sufficient calcium ions into the stomach. As a result, a problem has been pointed out that the efficiency with which a calcium content is digested and absorbed in the stomach of such a chicken is significantly reduced. This may thin the shell of a laid egg, increasing the egg breakage rate. This increase in the egg breakage rate may lead to economic disadvantages such as reduction of the commercial value and increases in transportation costs.

Conventionally, feeds containing organic foods, such as cereal crops, as main ingredients are used as feeds given to domestic animals. To supply other nutrients, mineral contents are further mixed in such feeds. For example, addition of granular calcium carbonate as a mineral content to fortify shells of poultry eggs and bones of domestic animals is known. However, calcium carbonate has very low solubility in water and is not well absorbed in an acidic condition in the stomach.

Accordingly, feed additives are conventionally known the calcium content of which is converted to a form easily absorbed in an organism, as disclosed in Patent Documents 1 and 2. For example, Patent Document 1 discloses a feed additive obtained by reacting an acid and calcium. The feed additive disclosed in Patent Document 1 is obtained by adding a seashell containing calcium to an aqueous acid solution containing citric acid and acetic acid and aging the filtrate after dissolution. However, this feed additive is a liquid in nature. Therefore, when a domestic animal ingests a feed containing this feed additive, the calcium content is taken up in a dissolved state (calcium ions). Accordingly, there has been a problem that the calcium content is hardly retained in the stomach for a long period after ingestion and only a transient increase in the calcium concentration in an organism can be expected.

Patent Document 2 discloses a composition treated with lactic acid. The lactic acid-treated composition contains calcium lactate obtained by adding a lactic acid solution to egg shells containing calcium carbonate. However, the lactic acid-treated composition is constituted by one type of organic acid calcium, i.e., calcium lactate. When a domestic animal ingests this composition, the calcium content is hardly retained in the stomach for a long period after ingestion and only a transient increase in the calcium concentration in an organism can be expected as in Patent Document 1.

Because of such a transient increase in the calcium ion concentration in an organism, effective calcium fortification cannot be expected in domestic animals during the breeding process. Further, there is a problem that the transient increase in the calcium ion concentration in an organism may place a burden on the kidneys of such domestic animals when an excess amount of calcium ions in the blood is excreted.
Patent Document 1: Japanese Laid-Open Patent Publication No. 2003-325112
Patent Document 2: Japanese Laid-Open Patent Publication No. 2007-45698

### Disclosure of the Invention

An objective of the present invention is to provide a calcium absorption enhancer that places no burden on the body of a human or a domestic animal to which a calcium compound is administered and can increase the efficiency with which a calcium content is absorbed in the body, after administration of a calcium compound, by supplying calcium ions in a sustained-released manner in the stomach or the like of the human or the domestic animal.

To achieve the foregoing objective, one aspect of the present invention provides a calcium absorption enhancer containing water-soluble calcium particles that can release calcium ions in a sustained-release manner in an aqueous solution as an active ingredient.

In the above-mentioned aspect, the above-mentioned water-soluble calcium particles preferably have a microscopic void structure in the surface thereof.

In the above-mentioned aspect, the above-mentioned microscopic void structure is preferably at least one structure selected from the group consisting of a capillary structure, a granular structure, and a spongy structure.

In the above-mentioned aspect, the above-mentioned water-soluble calcium particles are preferably obtained by treating a calcium-containing material with an acid.

In the above-mentioned aspect, the above-mentioned calcium-containing material is preferably a biological material.

In the above-mentioned aspect, the above-mentioned biological material is preferably at least one material selected from the group consisting of an egg shell, a seashell, an echinoderm shell, and an echinoderm spine.

In the above-mentioned aspect, the above-mentioned acid is preferably an organic acid.

In the above-mentioned aspect, the above-mentioned organic acid is preferably at least one acid selected from the group consisting of citric acid, acetic acid, lactic acid, formic acid, malic acid, propionic acid, butyric acid, gluconic acid, succinic acid, and valeric add.

In the above-mentioned aspect, the above-mentioned water-soluble calcium particles preferably have an average particle size of 0.1 to 10 mm.

In the above-mentioned aspect, the above-mentioned calcium absorption enhancer is preferably used for administration to domestic animals.

In the above-mentioned aspect, the above-mentioned calcium absorption enhancer is preferably used for administration to humans.

In the above-mentioned aspect, the above-mentioned water-soluble calcium particles are preferably mixture of two or more types of water-soluble calcium compounds having different solubility in water or an aqueous solution.

In the above-mentioned aspect, the above-mentioned water-soluble calcium compounds are preferably an organic acid calcium.

In the above-mentioned aspect, the above-mentioned organic acid calcium is preferably at least one compound selected from the group consisting of calcium citrate, calcium acetate, calcium lactate, calcium formate, calcium malate, calcium propionate, calcium butyrate, calcium gluconate, calcium succinate, and calcium valerate.

### Brief Description of the Drawings

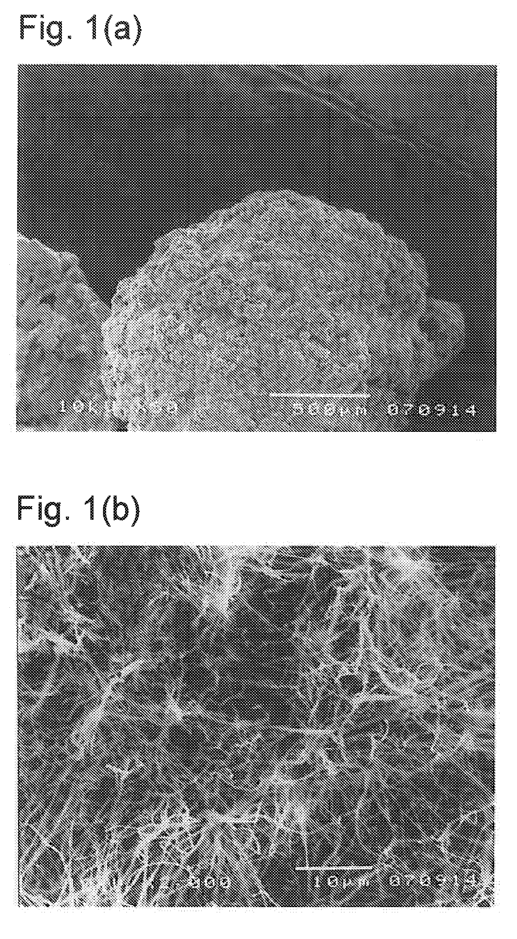

Fig. 1 provides electron micrographs showing the surface condition of the water-soluble calcium particle (1) obtained in Example 1, where (a) is a 50-times magnified electron micrograph of the water-soluble calcium particle (1) and (b) is a 2000-times magnified electron micrograph of the water-soluble calcium particle (1);

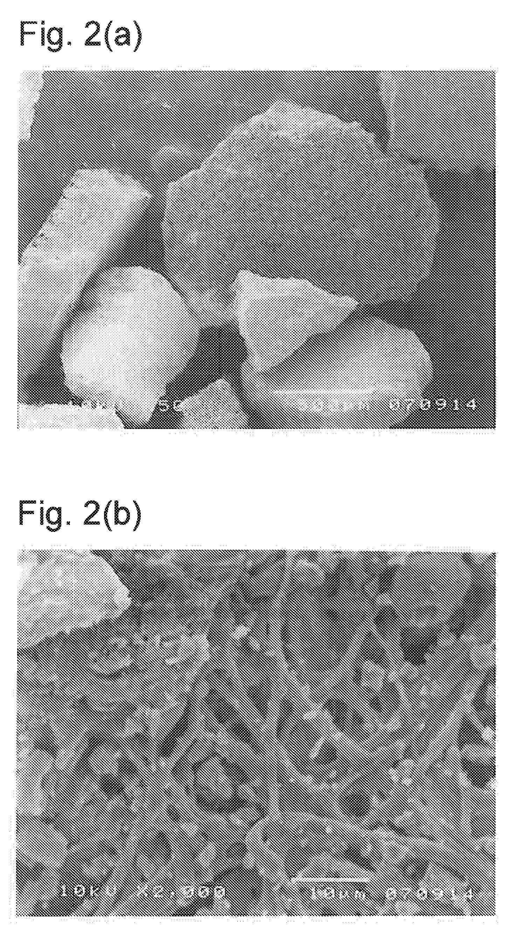

Fig. 2 provides electron micrographs showing the surface condition of the crushed egg shell particle (untreated egg shell particle) used in Example 1, where(a) is a 50-times magnified electron micrograph of the crushed egg shell particles, and (b) is a 2000-times magnified electron micrograph of the crushed egg shell particle;

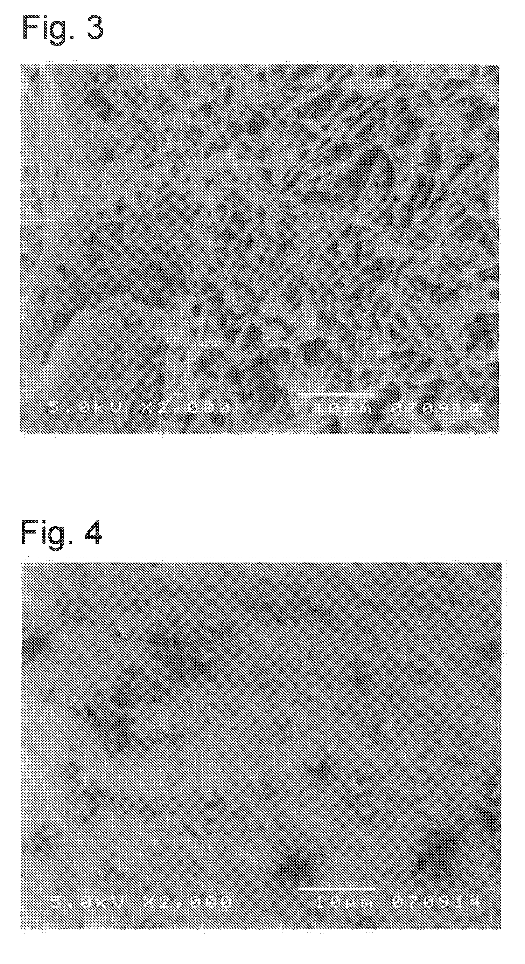

Fig. 3 is a 2000-times magnified electron micrograph showing the surface condition of the water-soluble calcium particle (2) obtained in Example 2;
Fig. 4 is a 2000-times magnified electron micrograph showing the surface condition of the crushed oyster shell particle (untreated oyster shell particle) used in Example 2;

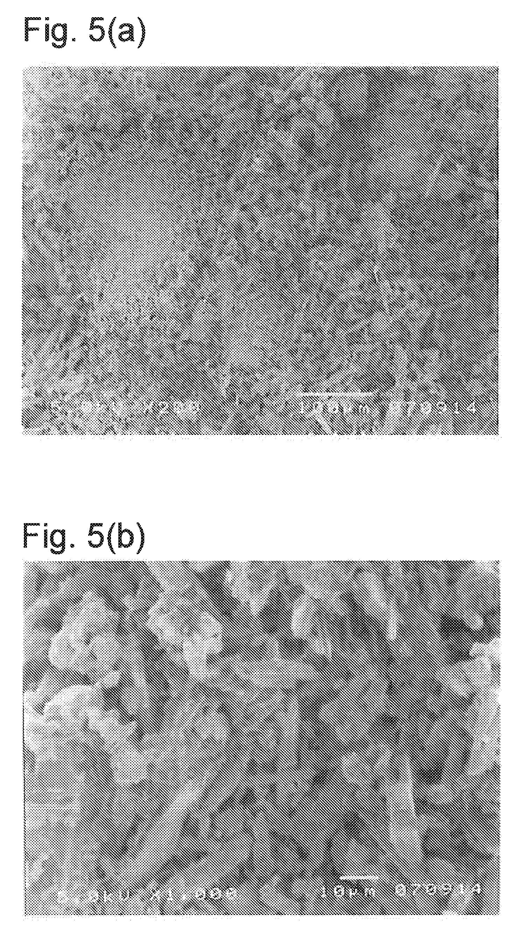

Fig. 5 provides electron micrographs showing the surface condition of the water-soluble calcium particle (3) obtained in Example 3, where (a) is a 200-times magnified electron micrograph of the water-soluble calcium particle (3) and (b) is a 1000-times magnified electron micrograph of the water-soluble calcium particle (3);

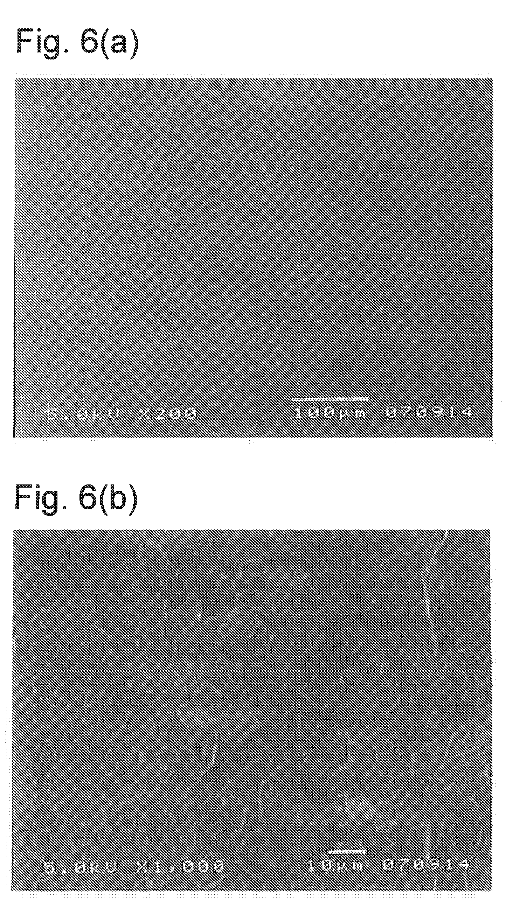

Fig. 6 provides electron micrographs showing the surface condition of the calcium carbonate particle (untreated calcium carbonate particle) used in Example 3, where (a) is a 200-times magnified electron micrograph of the untreated calcium carbonate particle and (b) is a 1000-times magnified electron micrograph of the untreated calcium carbonate particle;

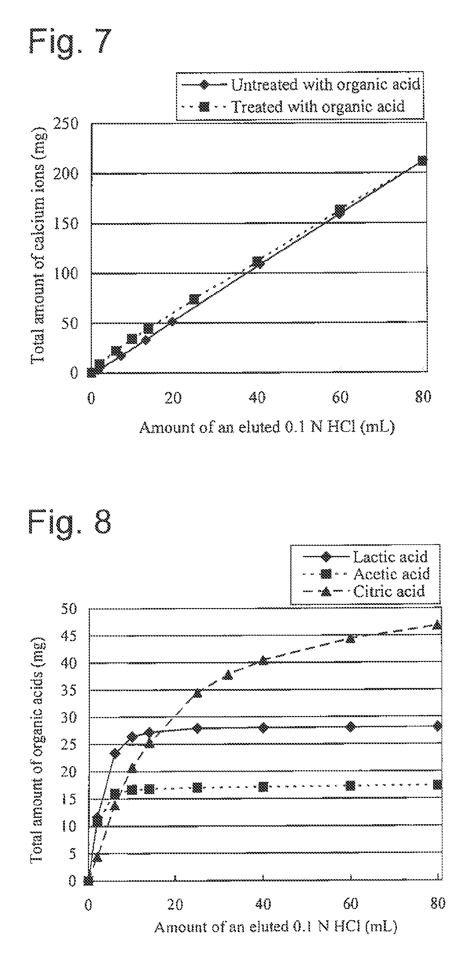

Fig. 7 is a graph showing the relationship between the amount of an eluted solvent (mL) and the amount of calcium ions (integrated value) when the organic acid-treated egg shell mixture (A) produced in Example 7 and crushed egg shell particles (control) were each used as a column filler, and 0.1 N aqueous HCl solution was used as a solvent;
Fig. 8 is a graph showing the relationship between the amount of an eluted solvent (mL) and the amount of each organic acid (integrated value) when the organic acid-treated egg shell mixture (A) produced in Example 7 was used, and 0.1 N aqueous HCl solution was used as a solvent;

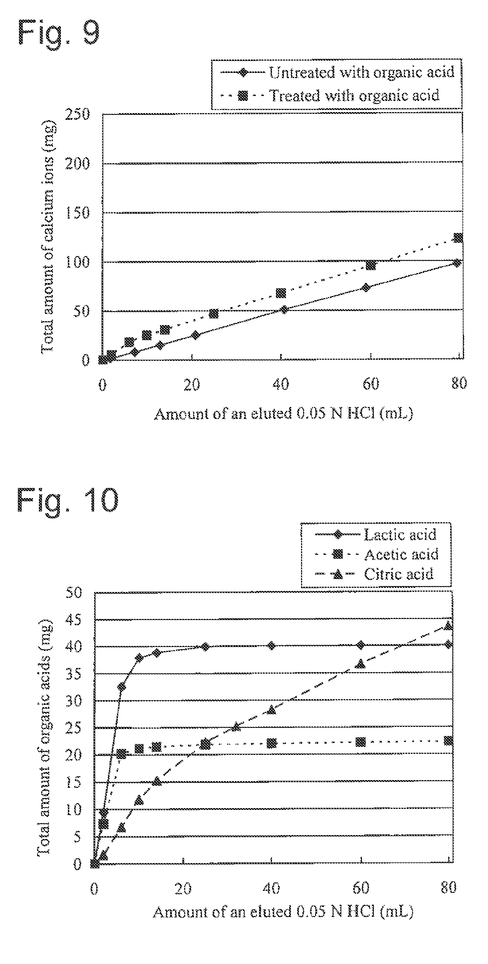

Fig. 9 is a graph showing the relationship between the amount of an eluted solvent (mL) and the amount of calcium ions (integrated value) when the organic acid-treated egg shell mixture (A) produced in Example 7 and crushed egg shell particles (control) were each used as a column filler, and 0.05 N aqueous HCl solution was used as a solvent;
Fig. 10 is a graph showing the relationship between the amount of an eluted solvent (mL) and the amount of each organic add (integrated value) when the organic acid-treated egg shell mixture (A) produced in Example 7 was used, and 0.05 N aqueous HCl solution was used as a solvent;

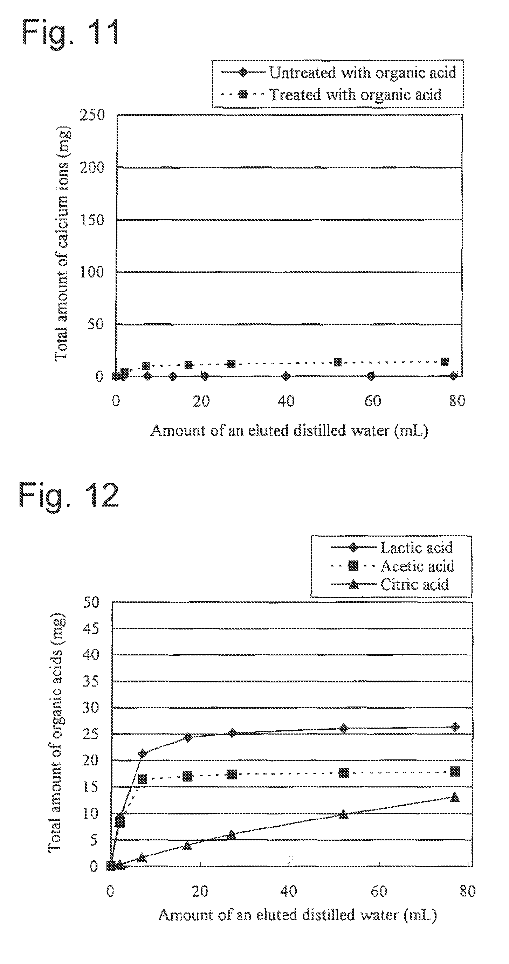

Fig. 11 is a graph showing the relationship between the amount of an eluted solvent (mL) and the amount of calcium ions (integrated value) when the organic acid-treated egg shell mixture (A) produced in Example 7 and crushed egg shell particles (control) were each used as a column filler, and distilled water was used as a solvent;
Fig. 12 is a graph showing the relationship between the amount of an eluted solvent (mL) and the amount of each organic acid (integrated value) when the organic acid-treated egg shell mixture (A) produced in Example 7 was used, and distilled water was used as a solvent;

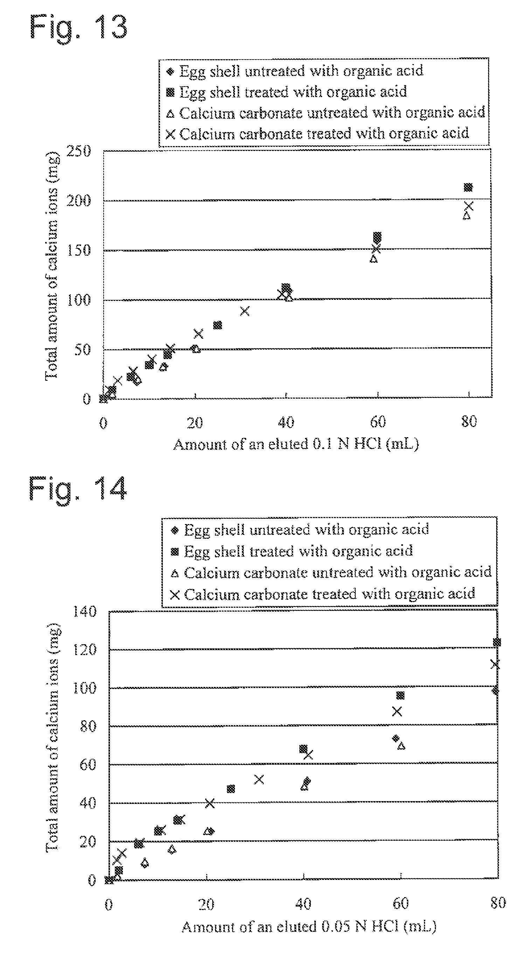

Fig. 13 is a graph showing the relationship between the amount of an eluted solvent (mL) and the amount of calcium ions (integrated value) when the organic acid-treated calcium carbonate particle mixture (B) produced in Example 14, untreated calcium carbonate particles (control), the organic acid-treated egg shell mixture (A) produced in Example 7, and untreated crushed egg shell particles (control) were each used as a column filler, and 0.1 N aqueous HCl solution was used as a solvent;
Fig. 14 is a graph showing the relationship between the amount of an eluted solvent (mL) and the amount of calcium ions (integrated value) when the organic acid-treated calcium carbonate particle mixture (B) produced In Example 14, untreated calcium carbonate particles (control), the organic acid-treated egg shell mixture (A) produced in Example 7, and untreated crushed egg shell particles (control) were each used as a column filler, and 0.05 N aqueous HCl solution was used as a solvent;

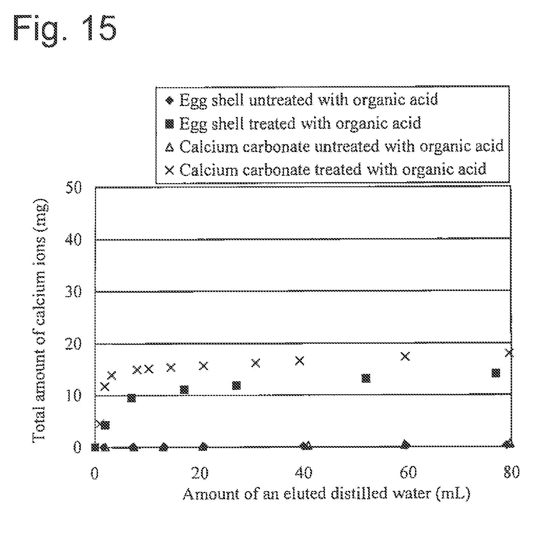

Fig. 15 is a graph showing the relationship between the amount of an eluted solvent (mL) and the amount of calcium ions (integrated value) when the organic acid-treated calcium carbonate particle mixture (B) produced in Example 14, untreated calcium carbonate particles (control), the organic acid-treated egg shell mixture (A) produced in Example 7, and untreated crushed egg shell particles (control) were each used as a column filler, and distilled water was used as a solvent;

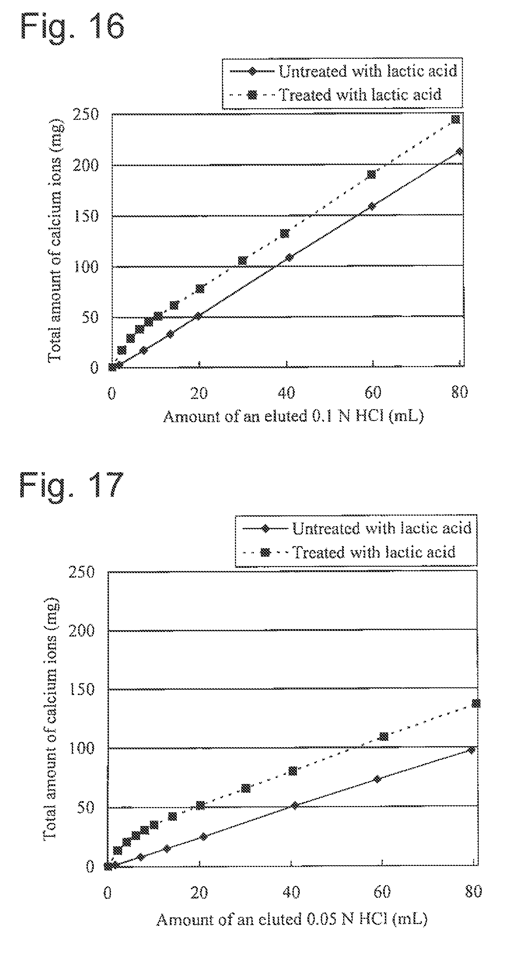

Fig. 16 is a graph showing the relationship between the amount of an eluted solvent (mL) and the amount of calcium ions (integrated value) when the water-soluble calcium particles (4) produced in Example 4 by treatment with lactic acid and crushed egg shell particles (control) were each used as a column filler, and 0.1 N aqueous HCl solution was used as a solvent;
Fig. 17 is a graph showing the relationship between the amount of an eluted solvent (mL) and the amount of calcium ions (integrated value) when the water-soluble calcium particles (4) produced in Example 4 by treatment with lactic acid and crushed egg shell particles (control) were each used as a column filler, and 0.05 N aqueous HCl solution was used as a solvent;

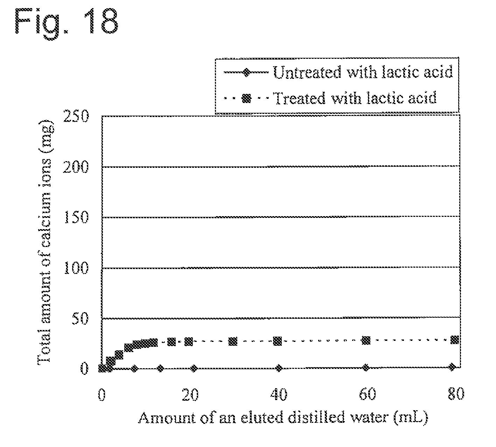

Fig. 18 is a graph showing the relationship between the amount of an eluted solvent (mL) and the amount of calcium ions (integrated value) when the water-soluble calcium particles (4) produced in Example 4 by treatment with lactic acid and crushed egg shell particles (control) were each used as a column filler, and distilled water was used as a solvent;

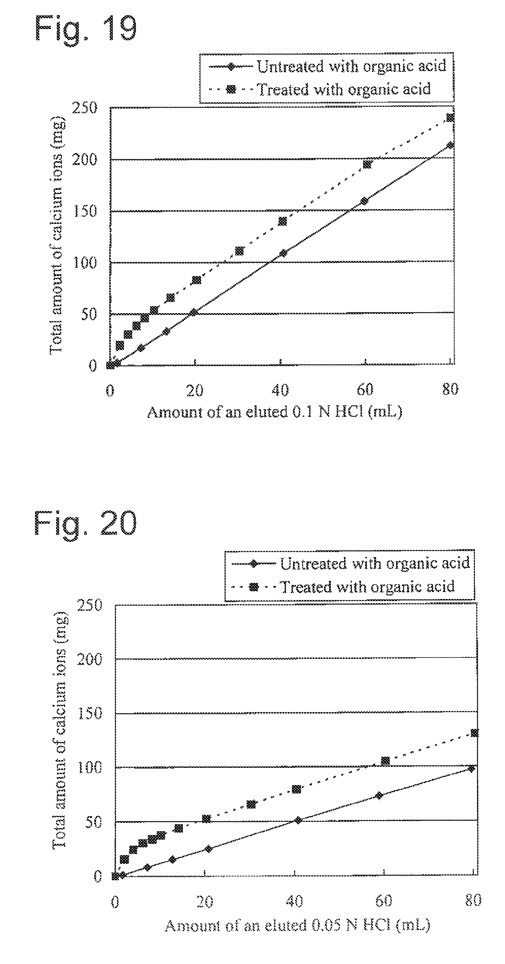

Fig. 19 is a graph showing the relationship between the amount of an eluted solvent (mL) and the amount of calcium ions (integrated value) when the water-soluble calcium particles (10) produced in Example 21 by treatment with an organic acid mixture and crushed egg shell particles (control) were each used as a column filler, and 0.1 N aqueous HCl solution was used as a solvent;
Fig. 20 is a graph showing the relationship between the amount of an eluted solvent (mL) and the amount of calcium ions (integrated value) when the water-soluble calcium particles (10) produced in Example 21 by treatment with an organic acid mixture and crushed egg shell particles (control) were each used as a column filler, and 0.05 N aqueous HCl solution was used as a solvent; and

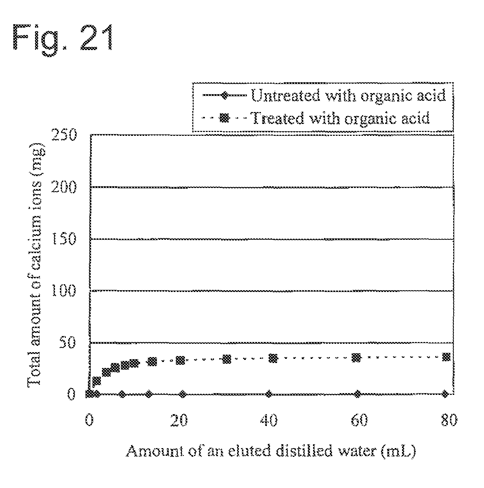

Fig. 21 is a graph showing the relationship between the amount of an eluted solvent (mL) and the amount of calcium ions (integrated value) when the water-soluble calcium particles (10) produced in Example 21 by treatment with an organic acid mixture and crushed egg shell particles (control) were each used as a column filler, and distilled water was used as a solvent.

### Best Mode for Carrying Out the Invention

Hereafter, specific embodiments of the calcium absorption enhancer of the present invention will be described in detail.

The calcium absorption enhancer of the present embodiment contains water-soluble calcium particles as an active ingredient. Here, the term used in the present specification "calcium absorption enhancer" includes materials that can be digested in the stomach or the like of a human or a domestic animal belonging to mammals or birds, produce calcium ions by this digestion, and convert the calcium ions into a form that can be absorbed in the organism as a nutrient. Further, "enhancement of calcium absorption" or "enhancement of ability of absorbing a calcium content" means to provide an environment that enables calcium ions produced by digestion to be absorbed in a large quantity or over a long period of time in the animal.

The water-soluble calcium particles used in the present embodiment can release calcium ions in an aqueous solution in a sustained-release manner.

The aqueous solution is not particularly limited and examples thereof include acidic aqueous solutions and neutral aqueous solutions. Examples of acidic aqueous solutions include digestive juice in the stomach of a human or a domestic animal, a mixture of the digestive juice in the stomach and water, a mixture of digestive juice in the stomach and a drink or a food ingested by a human or a domestic animals (accumulation in the stomach after a human or a domestic animal ingests a food or a drink). Examples of neutral aqueous solutions include digestive juice in the stomach of a human or a domestic animal, a mixture of the digestive juice in the stomach and water, and digestive juice neutralized when a mixture of the digestive juice in the stomach and a drink or a food ingested by a human or a domestic animal is transported from the stomach to the duodenum. The pH of an aqueous solution is not particularly limited and is preferably pH 1 to 7, more preferably pH 3 to 7.

The water-soluble calcium particles used in the present embodiment do not transiently release calcium ions In an aqueous solution but can release calcium ions while gradually dissociating the calcium ions. Specifically, the water-soluble calcium particles have a predetermined (ion) dissociation constant. The pK_{d} of the water-soluble calcium particles is preferably 1 to 7, more preferably 3 to 5.

Examples of the water-soluble calcium particles that can release calcium ions in an aqueous solution in a sustained-release manner include water-soluble calcium particles having a microscopic void structure and water-soluble calcium particles comprising a mixture of two or more types of water-soluble calcium compounds having different solubility in water or an aqueous solution. Hereafter, the calcium absorption enhancer containing water-soluble calcium particles having a microscopic void structure is referred to as a "first calcium absorption enhancer," and the calcium absorption enhancer containing water-soluble calcium particles comprising a mixture of two or more types of water-soluble calcium compounds having different solubility in water or an aqueous solution is referred to as a "second calcium absorption enhancer."

First, the first calcium absorption enhancer will be described. The first calcium absorption enhancer has a microscopic void structure in the surface of water-soluble calcium particles, which are active ingredient. Here, the "microscopic void structure" used in the present embodiment refers to the surface condition of water-soluble calcium particles that can be observed with magnifying observation means such as a microscope, for example, an electron microscope. Furthermore, the microscopic void structure also refers to a three-dimensional structure constituting predetermined irregularities and a structure showing a state where smoothness of the surface of the water-soluble calcium particles is lost. The water-soluble calcium particles, which are the active ingredient of the first calcium absorption enhancer, has a surface in a porous form due to this microscopic void structure. With this porous form, the water-soluble calcium particles can pass or retain a liquid, for example, a water content, and air.

The microscopic void structure may have a different three-dimensional structure depending on, for example, the type of a raw material constituting the water-soluble calcium particles and the type of acid treatment described later. Examples of the microscopic void structure include a capillary structure, a granular structure, and a spongy structure.

A capillary structure can be obtained when, for example, an egg shell powder is used as a raw material constituting the water-soluble calcium particles. Preferred examples of the capillary structure include many longitudinal structures, such as a structure in which a cylindrical, polygonal columnar, needle-like, hourglass-shaped, or inverse biconical structure is connected to the end and/or the body of another longitudinal structure at the end and/or the body thereof to form a network aggregation. Many voids are formed between the aggregates. The axial length of a longitudinal structure constituting such a capillary structure is not particularly limited and is preferably 1 to 30 µm, more preferably 5 to 20 µm. The periaxiai diameter of the longitudinal structure is not particularly limited and is preferably 0.1 to 4 µm, more preferably 0.5 to 2 µm.

A granular structure can be obtained when, for example, chemically synthesized calcium carbonate with relatively high purity is used as a calcium-containing material. The granular structure is preferably an aggregate of many polygonal columnar or polygonal needle-like microscopic granules. The axial length of a microscopic granule constituting a granular structure is not particularly limited and is preferably 2 to 30 µm, more preferably 5 to 25 µm. The periaxial diameter of the microscopic granule is not particularly limited and is preferably 1 to 8 µm, more preferably 2 to 5 µm.

A spongy structure can be obtained when, for example, a seashell is used as a calcium-containing material. The spongy structure is preferably a structure body having many pores therein. The diameter of a pore constituting a spongy structure (diameter of a hypotheticl circle inscribed in the pore) is not particularly limited and is preferably 0.2 to 15 µm, more preferably 2 to 10 µm.

The water-soluble calcium particles in the first calcium absorption enhancer of the present embodiment can be obtained by, for example, treating a calcium-containing material with an acid.

Examples of the calcium-containing material used In acid treatment include calcium carbonate and materials containing calcium carbonate. As the calcium-containing material, either a natural material or an artificially synthesized material can be used. As the calcium-containing material, a natural material is preferably used from viewpoints of easy availability and safety to an organism.

Examples of natural materials include mineral-derived materials and biological materials. Examples of mineral-derived calcium-containing materials include limestone, marble, stalactite, and chalk. Examples of biological calcium-containing materials include egg shells, seashells, echinoderm shells, echinoderm spines, coral skeletons, and vertebrate bones. As the naturally occurring calcium-containing materials, biological calcium-containing materials are preferred from a viewpoint of easy availability. In particular, egg shells, seashells, echinoderm shells, and echinoderm spines are more preferably used from a viewpoint that industrial waste can be effectively utilized. Egg shells are particularly preferred because they contain zinc abundantly as a nutrient in addition to a calcium content and further can easily be crushed in a predetermined particle size.

Birds for obtaining an egg shell are not particularly limited and any of breeds for egg, breeds for meat, and breeds for both egg and meat can be used. Examples of types of birds for obtaining an egg shell include chickens, quails, turkeys, ducks, ostriches, and geese. Examples of chicken include Leghorn and Minorca, which are breeds for egg, White Cornish and White Rock, which are breeds for meat, and Plymouth Rock and Rhode Island Red, which are breeds for both egg and meat.

Types of shellfish for obtaining a seashell are not particularly limited and preferred examples include oysters, scallops, Japanese littlenecks, turban shells, fresh water clams, and abalones from a viewpoint of easy availability. Types of echinoderms are not particularly limited. Types that contain calcium carbonate abundantly in a shell or a spine thereof are preferred, and examples thereof include sea urchins.

A calcium-containing material is preferably washed, sterilized, and disinfected In advance by means known to those skilled in the art, if necessary.

A calcium-containing material is preferably prepared in a predetermined particle size range and/or particle size distribution in advance by means known to those skilled in the art before treatment with an acid. At this time, the average particle size of the calcium-containing material can be suitably selected depending on the type, age, and body weight of a human or a domestic animal ingesting the calcium absorption enhancer of the present embodiment. The average particle size of a calcium-containing material is preferably 0.1 to 10 mm, more preferably 0.5 to 5 mm, further preferably 1 to 3 mm. When the average particle size is smaller than 0.1 mm, organic acid calcium is rapidly dissolved upon digestion in the stomach and the sustained-release effect may not be achieved. On the other hand, when the average particle size exceeds 10 mm, oral ingestion may be difficult depending on the type, size, and age of a human or a domestic animal. The average particle size of the calcium-containing material in the above-mentioned range can be achieved by, for example, passing through a sieve having a mesh with a specific size to prepare particles with a predetermined particle size distribution.

As an acid for treating calcium-containing material, any of chemically synthesized acids, naturally occurring acids, acids of combination thereof, and acid materials containing an acid may be used. Acids may be either inorganic or organic, but organic adds, with which water-soluble calcium particles having a higher sustained-release effect can be finally obtained, are preferably used. Examples of inorganic acids include mineral adds. Examples of mineral acids include hydrochloric acid, nitric add, and sulfuric acid. Examples of organic acids include acids comprising a hydrocarbon group at least one carbon of which may be substituted with a hydroxyl group and a carboxylic add group. Preferred examples of organic acids include unsubstituted carboxylic acids comprising a hydrocarbon group and a carboxylic acid group, such as formic add, acetic add, propionic acid, butyric acid, succinic acid, and valeric acid, hydroxy monocarboxylic adds, such as lactic acid and gluconic add, hydroxy dicarboxylic acids, such as malic acid, and hydroxy tricarboxylic acids, such as citric acid.

When a chemically synthesized organic acid is used, the organic acid is preferably used in the form of an aqueous solution, in which the organic acid is mixed with water. This is because this form is easy to handle and has favorable reactivity with a calcium-containing material. The concentration of an aqueous solution of an organic add used in the present embodiment is not particularly limited because the concentration varies depending on the production conditions, such as the type of an organic acid to be used, the size of water-soluble calcium particles to be produced, and the type of a calcium-containing material. For example, the concentration of an aqueous organic acid solution is preferably 10 mM or higher, more preferably 150 mM to 10 M. When the concentration of an aqueous solution of an organic add used is lower than 10 mM, the reactivity with a calcium-containing material is decreased. Therefore, intended water-soluble calcium particles may not be produced.

Examples of naturally occurring organic acid materials include foods containing citric acid, foods containing acetic acid, and foods containing lactic acid. Examples of foods containing citric acid include citruses, pickled plums, and vinegar from plum. Examples of foods containing acetic acid include apple vinegar, wine, and fermentation products obtained by fermenting agricultural crops, such as malt, rice, and potato, by acetic acid-producing bacteria. Examples of foods containing lactic acid include yogurt, cheese, pickles, and butter.

The first calcium absorption enhancer containing water-soluble calcium particles having a microscopic void structure can be obtained by bringing a calcium-containing material and an acid into contact. The method for bringing a calcium-containing material and an acid into contact is, for example, as follows. Examples of the method for treatment of a calcium-containing material with an acid include a method of spraying a calcium-containing material with one or more types of adds or an aqueous solution of the acids by means known to those skilled in the art and a method of immersing a calcium-containing material in an immersion bath containing one or more types of adds or an aqueous solution of the acids. The concentration of an acid used in acid treatment can be suitably selected depending on the acid treatment conditions. When the spraying method is used, a known method can be suitably employed. When the immersion method is used, the immersion time is not particularly limited because it varies depending on the immersion conditions, such as the type of a calcium-containing material used and the average particle size. The immersion time is preferably 0.05 to 48 hours, more preferably 0.1 to 1 hour. The temperature of the immersion bath is not particularly limited for the same reason as described above. The temperature of the immersion bath is adjusted preferably to 4°C to 160°C, more preferably 20°C to 70°C from a viewpoint that a reaction can be appropriately promoted. After a calcium-containing material is treated with an acid, the obtained water-soluble calcium particles may be subjected to drying treatment, if necessary.

The obtained water-soluble calcium particles may have a microscopic void structure over the whole particle surface or over at least a part of the particle surface. The particle surface includes not only the particle surface, but also a portion in the vicinity of the particle surface.

Hereafter, the second calcium absorption enhancer will be described. The second calcium absorption enhancer contains, as an active ingredient, a mixture of two or more types of water-soluble calcium compounds having different solubility (concentration of a solute in a saturated solution) in water or an aqueous solution as water-soluble calcium particles that can release calcium ions in water or an aqueous solution in a sustained-release manner.

Examples of an aqueous solution include acidic aqueous solutions and neutral aqueous solutions. Examples of acidic aqueous solutions include digestive juice in the stomach of a human or a domestic animal, a mixture of the digestive juice in the stomach and water, a mixture of digestive juice in the stomach and a drink or a food ingested by a human or a domestic animal (accumulation in the stomach after a human or a domestic animal ingests a food or a drink). Examples of neutral aqueous solutions include digestive juice in the stomach of a human or a domestic animal, a mixture of the digestive juice in the stomach and water, and digestive juice neutralized when a mixture of the digestive juice in the stomach and a drink or a food ingested by a human or a domestic animal is transported from the stomach to the duodenum.

Examples of water include pure water, ion exchange water, tap water, and other waters that can be ingested by humans or domestic animals.

Solubility of a water-soluble calcium compound contained in the second calcium absorption enhancer is not particularly limited. When solubility of the water-soluble calcium compound is expressed with a percent of the amount (g) of the water-soluble calcium compound, which is a solute, to 100 g of a solvent at 25°C, for example, the solubility is preferably 0.001 % to 40% to water at 25°C, more preferably 0.002% to 10%.

In the second calcium absorption enhancer of the present embodiment, two or more types of such water-soluble calcium compounds are used in combination. For example, three or more types, four or more types, and five or more types of water-soluble calcium compounds may be used in combination. Here, the water-soluble calcium compounds are preferably used in combination depending on the types thereof, so that solubility in water or an aqueous solution should be different from each other. This is because, after ingestion by a human or a domestic animal, calcium ions are eluted in the stomach or the like In a stepwise manner due to this difference in solubility. This prevents transient increases of the calcium ion concentration in blood.

In the second calcium absorption enhancer of the present embodiment, the degree of the difference in solubility between two or more water-soluble calcium compounds in water-soluble calcium particles used in one calcium absorption enhancer is not particularly limited. In other words, among combinations of water-soluble calcium compounds contained in one calcium absorption enhancer, a combination between a water-soluble calcium compound having the lowest solubility (Yₘₘ [%]) and a water-soluble calcium compound having the highest solubility (Yₘₐₓ [%]) should be selected, and the difference in each solubility Y_{dif} (Yₘₐₓ - Yₘᵢₙ [%]) is not particularly limited. This is because the preferable value varies depending on, for example, the type, build, age, and/or the condition in the stomach (water content, presence or absence of other ingested food, and the amount thereof) of the ingesting human or domestic animal. In the second calcium absorption enhancer of the present embodiment, when the degree of difference in solubility between water-soluble calcium compounds is expressed with a percent of the amount of the water-soluble calcium compound (g) to 100 g of a solvent, for example, it is preferably 0.8% to 40% to water at 25°C, more preferably 4% to 40%. When Y_{dif}, the difference in solubility between water-soluble calcium compounds to water at 25°C, is less than 0.8%, the water-soluble calcium compounds are dissolved in the stomach of a human or a domestic animal in a short time, increasing the calcium ion concentration in blood transiently. Or, since water-soluble calcium compounds are not dissolved, they may be hardly absorbed in the body. On the other hand, when Y_{dif}, the difference in solubility between water-soluble calcium compounds to water at 25°C, exceeds 40%, calcium ions may be eluted in the stomach intermittently or in a stepwise manner with a rapid change between steps. When Y_{dif}, the difference in solubility between water-soluble calcium compounds to water at 25°C, is within the above-mentioned range, calcium ions in the stomach are eluted in a sustained-release manner. Therefore, absorption of calcium ions in an organism can be further improved.

As water-soluble calcium particles used in the second calcium absorption enhancer of the present embodiment, commercially available products for food and drink are preferably used. This is because these products are easily available and safety thereof has been confirmed in ingestion by humans or domestic animals. The water-soluble calcium compound is preferably an organic add calcium itself or particles containing the organic acid calcium.

Organic acid calcium may be either naturally occurring or artificially or chemically synthesized. Examples of organic acid calcium used in the second calcium absorption enhancer of the present embodiment include calcium citrate (solubility, 0.045% in water at 25°C), calcium acetate (solubility, 40% in water at 25°C), calcium lactate (solubility, 4.8% in water at 25°C), calcium formate (solubility, 16.6% in water at 20°C), calcium malate (solubility, 0.92% in water at 20°C), calcium propionate (solubility, 26% in water at 20°C), calcium butyrate (solubility, 18.2% in water at 25°C), calcium gluconate (solubility, 3% in water at 25°C), calcium succinate, and calcium valerate. In the second calcium absorption enhancer of the present embodiment, for example, (1) a combination of calcium acetate and calcium citrate, (2) a combination of calcium lactate and calcium citrate, (3) a combination of calcium acetate and calcium lactate, (4) a combination of calcium acetate and calcium formate, (5) a combination of calcium formate and calcium citrate, (6) a combination of calcium formate and calcium lactate, (7) a combination of calcium acetate, calcium lactate, and calcium citrate, (8) a combination of calcium acetate, calcium lactate, and calcium formate, (9) a combination of calcium acetate, calcium citrate, and calcium formate, (10) a combination of calcium lactate, calcium citrate, and calcium formate, and (11) a combination of calcium acetate, calcium lactate, calcium citrate, and calcium formate are preferably used because these combinations can be easily mass-produced and obtained relatively easily.

In the second calcium absorption enhancer of the present embodiment, water-soluble calcium particles contained as an active ingredient is used in the form of a mixture of two or more types of water-soluble calcium compounds. Here, "a mixture of two or more types of water-soluble calcium compounds" in the present specification includes any of the following:
(a1) when one water-soluble calcium particle contains one type of water-soluble calcium compound, two or more types of water-soluble calcium particles are combined (a mixture of different types of water-soluble calcium particles);
(a2) one water-soluble calcium particle contains two or more types of water-soluble calcium compounds (a single water-soluble calcium particle comprising two or more components); and
(a3) combinations of the above,
   and the water-soluble calcium particles are any of the following:
(b1) water-soluble calcium compounds itself;
(b2) a mixture of water-soluble calcium compounds and other components; and
(b3) combination of the above.

In the second calcium absorption enhancer of the present embodiment, when the water-soluble calcium particles are characterized by both of any of such (a1) to (a3) and any of such (b1) to (b3). Thus, after ingestion thereof by a human or a domestic animal, the calcium content is not transported from the stomach to the intestines in a shorter time and can be retained in the stomach for a longer time as compared with ingestion of an aqueous calcium solution in a liquid state. Time for supplying calcium ions In the body can be extended by increasing the retention time of the calcium content in the stomach.

In the second calcium absorption enhancer of the present embodiment, water-soluble calcium particles are preferably prepared in a predetermined particle size range and/or with a particle size distribution from viewpoints of increasing a calcium content in the stomach in a sustained-release manner and facilitating oral ingestion by a human or a domestic animal.

For example, in the second calcium absorption enhancer, the average particle size of water-soluble calcium particles is preferably 0.1 to 10 mm, more preferably 1 to 5 mm, further preferably 2 to 3 mm. When the average particle size is smaller than 0.1 mm, water-soluble calcium particles are rapidly dissolved upon digestion in the stomach, and calcium ions may not be eluted efficiently in a sustained-release manner. On the other hand, when the average particle size exceeds 10 mm, oral ingestion may be difficult depending on the type, size, and age of a human or a domestic animal. The average particle size in the above-mentioned range can be achieved by, for example, passing through a sieve with a mesh having a specific size to prepare particles with a predetermined particle size distribution.

The second calcium absorption enhancer of the present embodiment can be produced by, for example, using the following two types of methods. In the first example, first, two or more types of organic acids are used and each organic acid is brought into contact with each calcium-containing material to generate one organic acid calcium, and then all organic acid calcium are mixed. In the second example, a mixture of two or more types of organic adds is brought into contact with a calcium-containing material to generate two or more types of organic acid calcium together.

As a method for producing the second calcium absorption enhancer, the first example will be described. As organic acids used in the first example, any of chemically synthesized organic acids, naturally occurring organic acids, and organic acid materials containing a naturally occurring organic acid may be used.

Examples of organic acids used in the first example include acids comprising a hydrocarbon group at least one carbon of which may be substituted with a hydroxyl group and a carboxylic acid group. Preferred examples of organic acids include unsubstituted carboxylic acids comprising a hydrocarbon group and a carboxylic acid group, such as formic acid, acetic acid, propionic acid, butyric acid, succinic acid, and valeric acid, hydroxy monocarboxylic acids, such as lactic acid and gluconic acid, hydroxy dicarboxylic acids, such as malic acid, and hydroxy tricarboxylic acid, such as citric acid.

When a chemically synthesized organic acid is used in the first example, the organic acid is preferably used in the form of an aqueous solution. This is because this form is easy to handle and has favorable reactivity with a calcium-containing material. The concentration of an aqueous solution of an organic acid used in the first example is not particularly limited because the concentration varies depending on the production conditions, such as the type of an organic acid to be used, the size of water-soluble calcium particles to be produced, and the origin of a calcium-containing material. For example, the concentration of an aqueous organic acid solution is preferably 10 mM or higher, more preferably 150 mM to 10 M. When the concentration of an aqueous solution of an organic acid used is lower than 10 mM, the reactivity with a calcium-containing material is decreased. Therefore, intended water-soluble calcium particles may not be produced.

Examples of an organic acid material containing a naturally occurring organic acid include foods containing citric acid, foods containing acetic acid, and foods containing lactic acid. Examples of foods containing citric acid include citruses, pickled plum, and vinegar from plum. Examples of foods containing acetic acid include apple vinegar, wine, and fermentation products obtained by fermenting agricultural crops, such as malt, rice, and potato, by acetic acid-producing bacteria. Examples of foods containing lactic acid include yogurt, cheese, pickles, and butter.

Examples of a calclum-containing material used in the first example include calcium carbonate itself and a material containing calcium carbonate. The calcium carbonate may be either naturally occurring or chemically synthesized. Naturally occurring calcium carbonate materials are preferably used because they are abundant in nature and can be easily obtained.

Examples of naturally occurring calcium carbonate materials include mineral-derived calcium carbonate materials and biological calcium carbonate materials. Examples of mineral-derived calcium carbonate materials include limestone, marble, stalactite, and chalk. Examples of biological calcium carbonate materials include egg shells, seashells, echinoderm shells, echinoderm spines, coral skeletons, and vertebrate bones. As the naturally occurring calcium carbonate materials, biological calcium carbonate materials are preferred from a viewpoint of easy availability. In particular, egg shells, seashells, echinoderm shells, and echinoderm spines are more preferably used from a viewpoint that industrial waste can be effectively utilized. Egg shells are particularly preferred because they contain zinc abundantly as a nutrient in addition to a calcium content and further can be easily crushed in a predetermined particle size. The types of shellfish used as seashells are not particularly limited. As shellfish for obtaining seashells, oyster, scallop, Japanese littleneck, turban shell, fresh water clam, and abalone are preferably used from a viewpoint of easy availability. The types of echinoderms are not particularly limited and types containing calcium carbonate in the shell or spine thereof are preferred, for example, sea urchin.

A calcium-containing material used in the first example is preferably prepared in advance in a predetermined particle size range and/or with a predetermined particle size distribution before being brought into contact with an organic acid by means known to those skilled in the art. At this time, the average particle size of a calcium-containing material is preferably 0.1 to 10 mm, more preferably 1 to 5 mm, further preferably 2 to 3 mm. The average particle size of the calcium-containing material can be suitably selected depending on the type, age, and body weight of a human or a domestic animal ingesting the second calcium absorption enhancer of the present embodiment. The average particle size of the calcium-containing material in the above-mentioned range can be achieved by, for example, passing through a sieve with a mesh having a specific size to prepare particles with a predetermined particle size distribution.

Examples of the method for bringing a calcium-containing material in the first example and an organic acid into contact include means known to those skilled in the art such as the following. Examples of such methods include a method of spraying a calcium-containing material with, for example, one organic acid or an aqueous solution of the organic acid and a method of immersing a calcium-containing material in an immersion bath comprising one organic acid or an aqueous solution of the organic acid prepared at a predetermined concentration. When the spraying method is used, the conditions are not particularly limited and can be arbitrarily selected by those skilled in the art. When the immersion method is used, the ratio of amounts of a calcium-containing material and an organic acid or an aqueous solution of the organic acid in an immersion bath can be arbitrarily selected by those skilled in the art. The immersion time is not particularly limited because it varies depending on immersion conditions, such as the type and the average particle size of a calcium-containing material immersed in the immersion bath. The immersion time is, for example, 0.05 to 48 hours, preferably 0.1 to 1 hour. The bath temperature of the immersion bath is not particularly limited for the same reason as described above. The bath temperature of the immersion bath is preferably 4°C to 160°C, more preferably 20°C to 70°C to promote a reaction appropriately. After an organic add and a calcium-containing material are brought into contact with each other, drying treatment may be performed, if necessary. By repeating the same method as described above using another type of an organic acid and a calcium-containing material, two or more types of water-soluble calcium particles with a layer consisting of one organic add calcium formed on the whole particle surface or a part thereof are produced separately. The particle surface includes not only the particle surface, but also a portion in the vicinity of the particle surface.

Subsequently, two or more types of water-soluble calcium particles produced as described above each containing one organic acid calcium are mixed into one. The water-soluble calcium particles can be mixed by means known to those skilled in the art. The mixing ratio of water-soluble calcium particles is not particularly limited. For example, when water-soluble calcium particles containing one organic add calcium and water-soluble calcium particles containing another organic acid calcium are mixed, a mixing ratio thereof may be, for example, even (1:1) using the volume as a reference or may be other ratios, such as in the range of 1:0.1 to 1:10.

As described above, the second calcium absorption enhancer of the present embodiment can be easily produced by using the first example.

Hereafter, the second example for producing the second calcium absorption enhancer of the present embodiment will be described. The second example is a method of bringing a mixture of two or more types of organic adds into contact with a calcium-containing material to produce two or more types of organic acid calcium together. As the calcium-oontaining material and organic acid used in the second example, the same calcium-containing materials and organic acids as in the first example can be used.

In the second example, two or more types of organic acids or an aqueous solution of the organic acids are mixed for use. The mixing ratio of each organic acid to be mixed is not particularly limited. For example, when one organic acid or an aqueous solution of the organic acid and another organic acid or an aqueous solution of the organic acid are mixed, the mixing ratio of these organic acids may be, for example, 1:1 (even) using the volume as a reference and may be other ratios, such as in the range of 1:0.1 to 1:10.

Examples of the method for bringing organic adds mixed as described above and a calcium-containing material into contact include means known to those skilled in the arts as in the first example, such as a spraying method and a method of immersing in an immersion bath. In the spraying method and the method of immersing in an immersion bath, the same means and conditions as in the above first example can be used. After organic acids and a calcium-containing material are brought into contact, drying treatment may be performed, if necessary. Thus, one water-soluble calcium particle with a layer consisting of two or more types of organic acid calcium formed on the whole particle surface or a part thereof is produced. The particle surface includes not only the particle surface, but also a portion in the vicinity of the particle surface. The water-soluble calcium particles obtained in the second example can be used as it is as the second calcium absorption enhancer of the present embodiment. As described above, by using the second example, the second calcium absorption enhancer of the present embodiment can be easily produced.

Hereafter, use of the calcium absorption enhancer of the present embodiment will be described.

The calcium absorption enhancer of the present embodiment is used as, for example, a food additive added to a food composition ingested by humans and a feed additive added to a feed composition fed to domestic animals. Examples of foods include general foods, health foods, functional foods, and supplements. Examples of domestic animals that can ingest the calcium absorption enhancer of the present embodiment include livestock, poultry, cultured fish, and land-dwelling pet animals. Among these, the calcium absorption enhancer of the present embodiment can be preferably applied to livestock, poultry, and land-dwelling pet animals for which calcium fortification is strongly demanded. Examples of livestock include horses, cows, pigs, sheep, goats, and donkeys. Examples of poultry include chickens, quails, turkeys, ducks, ostriches, and geese. Examples of chickens include Leghorn and Minorca, which are breeds for egg, broiler, which is a breed for meat, and Plymouth Rock and Rhode Island Red, which are breeds for both egg and meat. Examples of land-dwelling pet animals include dogs, cats, rabbits, hamsters, monkeys, myna birds, society finches, parakeets, long-tailed cocks, bantams, and pigeons.

First, the case where the calcium absorption enhancer of the present embodiment is used as a food additive added to a food composition ingested by humans will be described.

The calcium absorption enhancer of the present embodiment may be mixed with other appropriate arbitrary additives when added to a food composition. Examples of other additives include processed meat, general food materials, sugars, general food additives, surfactants, excipients, colorants, preservatives, coating aids, polyvinylpyrrolidone, oils, moisturizing agents, thickening agents, antiseptics, flavors, disinfectants, stabilizers, and combinations thereof. Examples of general food materials include rice, wheat, maize, potato, sweet potato, soybean, laminaria, Undaria pinnatifida, Gelidiaceae, and powders thereof. Examples of sugars include starch, starch syrup, milk sugar, glucose, fructose, sucrose, mannitol, lactose, dextrin, corn starch, sorbitol, and crystalline cellulose. Examples of general food additives include spices, sweeteners, edible oils, and vitamins. The content of other food additives in a food composition is not particularly limited so long as the effect of the calcium absorption enhancer of the present embodiment is not interfered.

Examples of the food composition include confectionaries, fruit products, vegetable products, animal meat products, fish products, delicacies, canned/jarred foods, mouth fresheners, and jellies. The content of the calcium absorption enhancer of the present embodiment in the food composition is not particularly limited and is preferably 0.1% to 100% by weight, more preferably 0.5% to 99.5% by weight, further preferably 2% to 99% by weight. The food composition can be produced by using techniques known to those skilled in the art.

The food composition containing the calcium absorption enhancer of the present embodiment may be labeled to the effect that it is used to increase the efficiency with which a calcium content is absorbed into the body because it has effect of providing calcium ions In the stomach in a sustained-release manner. Examples of the food composition with a label showing such a function include foods for specified health use and foods for special use. Examples of other function labels for the food composition include a label to the effect of being used to increase or improve the efficiency with which a calcium content is absorbed into the body, a label to the effect of being a food (or a food and a drink) for those who are concerned about bone health, a label to the effect of being used for preventing and/or improving osteoporosis, a label to the effect of being used for relieving or reducing a risk of osteoporosis, and a label to the effect of being used for promoting bone formation. It is sufficient that the label is expressed in a format so that the above-mentioned function can be substantially understood by users and, for example, the outer or inner package of a drink or a food, a commodity catalog, and a poster are labeled.

Hereafter, the case where the calcium absorption enhancer of the present embodiment is used as a feed additive added to a feed composition to be fed by domestic animals will be described. The calcium absorption enhancer of the present embodiment may be mixed with other appropriate arbitrary additives when added to a feed composition. As other additives, known additives can be suitably selected depending on the type, size, and age of domestic animals. Specific examples thereof include general feed materials, vegetable oil meal, animal feeds, bran, sugars, general feed additives, vitamins, minerals, amino acids, anti-oxidizing agents, enzymes, viable cell agents, sweeteners, emulsifiers, excipients, colorants, preservatives, coating aids, polyvinylpyrrolidone, oils, moisturizing agents, thickening agents, antiseptics, flavors, antibacterial agents, and stabilizers. Examples of general feed materials include crops, Gramineae feed crops, leguminous feed crops, potatoes, and powders thereof. Examples of crops include rice, wheat, maize, Milo, and millet. Examples of leguminous feed crops include alfalfa and clover. Examples of potatoes include potatoes and sweet potatoes. Examples of vegetable oil meal include soybean oil meal, rapeseed oil meal, corn gluten meal, corn jam meal, and sesame oil meal. Examples of animal feeds include fish meal, pork meal, chicken meal, soft roe, and feather meal. Examples of bran include rice bran oil meal, corn gluten feed, wheat bran, rice bran, and bean curd refuse. Examples of sugars include starch, starch syrup, milk sugar, glucose, fructose, sucrose, mannitol, lactose, dextrin, corn starch, sorbitol, and crystalline cellulose. Examples of general feed additives include spice, animal oils and fats, calcium phosphate, common salt, zedoary, turmeric, yeast, garlic powder, fermented milk powder, vegetable oils and fats, paprika extract, calcium carbonate, and corn steep liquor. Other feed additives in a feed composition are not particularly limited so long as the effect of the calcium absorption enhancer of the present embodiment is not interfered.

The content of the calcium absorption enhancer of the present embodiment in a feed composition is preferably 0.1% to 2% by weight, more preferably 0.5% to 1% by weight. The feed composition can be produced by techniques known to those skilled in the art.

The dose of the calcium absorption enhancer of the present embodiment can be suitably selected depending on the type, size, and age of an ingesting human or domestic animal. For example, when a human orally ingests a calcium absorption enhancer, the daily adult dose of the calcium absorption enhancer is preferably 5 to 50 mg/kg body weight, more preferably 10 to 30 mg/kg body weight In terms of a water-soluble calcium particle solid (dry weight). The number of times of ingestion of the calcium absorption enhancer is not particularly limited and may be once daily or two or more times daily. The method for administering the calcium absorption enhancer is not particularly limited and may be any of oral administration and enteral administration (methods for administering into the stomach or the intestine directly).

The present embodiment has the following advantages.
(1) When the calcium absorption enhancer of the present embodiment is administered to a human or a domestic animal, it is gradually eluted into the contents in the stomach containing gastric juice secreted from the stomach and an ingested water content. Therefore, the efficiency with which a calcium content is absorbed into the body can be increased, fortifying calcium efficiently.
(2) The calcium absorption enhancer of the present embodiment preferably has a microscopic void structure in the surface of the water-soluble calcium particles. This microscopic void structure enables the water-soluble calcium particles to pass or retain a liquid, for example, a water content, and air. Consequently, calcium ions are not transiently released, but gradually released from the water-soluble calcium particles into an aqueous solution. Therefore, the efficiency with which a calcium content is absorbed into the body can be increased, and calcium fortification can be achieved more efficiently.
(3) In the calcium absorption enhancer of the present embodiment, solid particles containing preferably two or more types of water-soluble calcium are used as water-soluble calcium particles. Therefore, even when an ingested water content is increased and pH in the stomach contents is increased (weak acidification), water-soluble calcium particles are gradually dissolved in the stomach without being completely dissolved at one time or in a short time due to differences in solubility between water-soluble calcium in the solid particles in the stomach contents.
   As a result, sustained release property of calcium ions in the stomach over a relatively long period of time can be maintained. Specifically, among two or more types of water-soluble calcium contained in the present embodiment, water-soluble calcium with high solubility in water or an aqueous solution release a high concentration of calcium ions immediately after the stage where they are brought into contact with stomach contents. On the other hand, since water-soluble calcium with low solubility elute only a small amount of calcium ions and the saturated dissolution amount is immediately reached, the calcium ion release is once suppressed at an early stage. However, as an organism absorbs calcium ions into the body, and the calcium ion concentration is decreased, continuous release of calcium ions in a small amount is expected.
(4) Since the calcium absorption enhancer of the present embodiment can provide calcium ions in an organism continuously over a long period without a rapid increase in the concentration, burdens on the kidneys of a human or a domestic animal can be reduced.
(5) The calcium absorption enhancer of the present embodiment can be preferably used as a feed additive for achieving calcium fortification effectively. In particular, poultry, for example, chickens significantly increase fluid intake in a high-temperature season in summer, and the gastric juice is diluted, decreasing the hydrochloric acid concentration in the gastric acid. Consequently, calcium absorption efficiency may be decreased. Even in such a case, however, continuous elution of calcium ions over a long period of time in the stomach of poultry can be expected by ingestion of the calcium absorption enhancer of the present embodiment. As a result, calcium ions are effectively absorbed into the body, the bones or the shell of laid eggs can be fortified. Based on the above advantages, the calcium absorption enhancer of the present embodiment prevents decreases in the strength of egg shells and decrease the egg breakage rate regardless of increases or decreases in fluid intake without affecting the environment for absorption of a calcium content In an organism.

The above-mentioned embodiment may be modified as follows.

In the above-mentioned embodiment, the calcium absorption enhancer can be used as an egg shell fortifier. The calcium absorbing ability of poultry ingesting the calcium absorption enhancer can be enhanced, and the shell of laid eggs can be fortified. Consequently, decreases in egg shell strength can be prevented, and thus the egg breakage rate can be decreased.

In the above-mentioned embodiment, the calcium absorption enhancer may be used as a bone fortifier. The calcium absorbing ability of a human or a domestic animal ingesting the calcium absorption enhancer can be enhanced, and the bone formation of the organism can be enhanced (bone fortification).

### Examples

Hereafter, the present embodiment will be specifically described with reference to the following examples. However, the scope of the present embodiment is not limited to these examples.

### <Example 1: Production of organic acid-treated egg shell particles (water-soluble calcium particles (1))>

As a calcium-containing material, an egg shell having an average particle size of 2 to 3 mm of a chicken (Leghorn) obtained by crushing in advance in a sealed bag using a blender (Zojirushi Corporation, model BM-FX08) and then passing through a sieve was used. As organic acids, a mixture solution of 15 g of 90% w/w lactic acid solution, 15 g of 10% w/w acetic acid solution, and 5 g of 3% w/w citric acid solution was used. The above-mentioned organic acid mixture solution (35% w/w to the egg shell) was gradually added to 100 g of the above-mentioned egg shell and mixed, and then the mixture was allowed to stand and reacted at room temperature for 5 hours. Subsequently, the mixture was left and dried at 60°C overnight to obtain 120 g of water-soluble calcium particles (1) containing calcium lactate (solubility in water at 25°C, 4.8%), calcium acetate (solubility in water at 25°C, 40%), and calcium citrate (solubility in water at 25°C, 0.045%).

The surface condition of the water-soluble calcium particles (1) obtained as described above was observed with an electron microscope (JEOL Ltd., JSM-5410LV). Fig. 1 shows electron micrographs of the water-soluble calcium particles (1). For comparison, the surface condition of crushed egg shell particles, which are calcium-containing material not subjected to acid treatment, was similarly observed with an electron microscope. Fig. 2 shows electron micrographs of the crushed egg shell particles.

As shown in Figs. 1 and 2, it was observed that the water-soluble calcium particles (1) (Fig. 1(a), 50-times magnification) had pores constituted by many irregularities in the particle surface eroded by organic acid treatment as compared with the untreated egg shell particles (Fig. 2(a), 50-times magnification). It was observed that the surface structure of the water-soluble calcium particles (1) (Fig. 1 (b), 2000-times magnification) had a clear change by organic acid treatment as compared with the untreated crushed egg shell particles (Fig. 2(b), 2000-times magnification). Specifically, it is shown that a reticular tissue structure constituting the egg shell was changed to a capillary structure by organic acid treatment, and even more gaps were formed around many longitudinal structures constituting the structure.

### <Example 2: Production of organic acid-treated oyster shell particles (water-soluble calcium particles (2))>

As a calcium-containing material, an oyster shell having an average particle size of 2 to 3 mm obtained by crushing in advance in a sealed bag using a blender (Zojirushi Corporation, model BM-FX08) and then passing through a sieve was used. As organic acids, a mixture solution of 15 g of 90% w/w lactic acid solution, 15 g of 10% w/w acetic acid solution, and 5 g of 3% w/w citric add solution was used. The above-mentioned organic acid mixture solution (35% w/w to the oyster shell) was gradually added to 100 g of the above-mentioned oyster shell and mixed, and then the mixture was allowed to stand and reacted at room temperature for 5 hours. Subsequently, the mixture was left and dried at 60°C overnight to obtain 120 g of water-soluble calcium particles (2) containing calcium lactate (solubility in water at 25°C, 4.8%), calcium acetate (solubility in water at 25°C, 40%), and calcium citrate (solubility in water at 25°C, 0.045%).

The surface condition of the water-soluble calcium particles (2) obtained as described above was observed with an electron microscope in the same manner as in Example 1. Fig. 3 shows an electron micrograph of the water-soluble calcium particles (2). For comparison, the surface condition of crushed oyster shell particles, which are calcium-containing material not subjected to acid treatment, was similarly observed with an electron microscope. Fig. 4 shows an electron micrograph of the crushed oyster shell particles.

As shown in Figs. 3 and 4, the water-soluble calcium particles (2) (Fig. 3) have a porous spongy structure formed in the particle surface eroded by organic acid treatment as compared with the untreated crushed oyster shell particles (Fig. 4).

### <Example 3: Production of organic acid-treated calcium carbonate particles (water-soluble calcium particles (3))>

A mixture solution of 15 g of 90% w/w lactic add solution, 15 g of 10% w/w acetic acid solution, and 5 g of 3% w/w citric acid solution was added to 100 g of calcium carbonate particles having an average particle size of 2 to 3 mm (feed grade) as a calcium-containing material in a flask and mixed, and then the mixture was allowed to stand and reacted at room temperature for 5 hours. Subsequently, the mixture was left and dried at 60°C overnight to obtain 120 g of water-soluble calcium particles (3) containing calcium lactate (solubility in water at 25°C, 4.8%), calcium acetate (solubility in water at 25°C, 40%), and calcium citrate (solubility in water at 25°C, 0.045%).

The surface condition of the water-soluble calcium particles (3) obtained as described above was observed with an electron microscope in the same manner as in Example 1. Fig. 5 shows electron micrographs of the water-soluble calcium particles (3). For comparison, the surface condition of calcium carbonate particles, which are calcium-containing material (calcium carbonate particles not subjected to acid treatment) was similarly observed with an electron microscope. Fig. 6 shows electron micrographs of the calcium carbonate particles not subjected to acid treatment.

As shown in Figs. 5 and 6, it was observed that the water-soluble calcium particles (3) (Fig. 5(a), 200-times magnification) had many granular structures formed in the particle surface eroded by organic acid treatment as compared with the calcium carbonate particles not subjected to acid treatment (Fig. 6(a), 200-times magnification), It was found that the surface structure of the water-soluble calcium particles (3) (Fig. 5(b), 1000-times magnification) had a clear change by organic acid treatment as compared with the calcium carbonate particles not subjected to acid treatment (Fig. 6(b), 1000-times magnification). Specifically, it is shown that a generally smooth surface constituting the calcium carbonate particles was changed to crystalline microscopic granules by organic add treatment, and many gaps were formed around the granules.

### <Example 4: Production of organic acid-treated egg shell particles (water-soluble calcium particles (4))>

As a calcium-containing material, an egg shell having an average particle size of 2 to 3 mm of a chicken (Leghorn) obtained by crushing in advance in a sealed bag using a blender (Zojirushi Corporation, model BM-FX08) and then passing through a sieve was used. An aqueous solution of 50 mmol lactic add was gradually added to 50 g of this egg shell and mixed, and then the mixture was allowed to stand and reacted at room temperature for 5 hours. Subsequently, the mixture was left and dried at 60°C overnight to obtain 55 g of water-soluble calcium particles (4) containing calcium lactate (solubility in water at 25°C, 4.8%).

### <Example 5: Production of organic acid-treated egg shell particles (water-soluble calcium particles (5))>

53 g of water-soluble calcium particles (5) containing calcium acetate (solubility in water at 25°C, 40%) was obtained in the same manner as in Example 4, except that an aqueous solution of 50 mmol of acetic acid was used instead of the aqueous lactic acid solution.

### <Example 6: Production of organic acid-treated egg shell particles (water-soluble calcium particles (6))>

60 g of water-soluble calcium particles (6) containing calcium citrate (solubility in water at 25°C, 0.045%) was obtained in the same manner as in Example 4, except that an aqueous solution of 50 mmol of citric acid was used instead of the aqueous lactic acid solution.

### <Example 7: Production of calcium absorption enhancer (organic acid-treated egg shell mixture (A))>

Equal amounts (0.5 g each) of the above-mentioned water-soluble calcium particles (4) obtained in Example 4, the above-mentioned water-soluble calcium particles (5) obtained in Example 5, the above-mentioned water-soluble calcium particles (6) obtained In Example 6, and egg shells were collected and mixed uniformly to obtain an organic acid-treated egg shell mixture (A). The difference in solubility Y_{dif} between the water-soluble calcium particles in the organic acid-treated egg shell mixture (A) was 39.955.

### <Example 8: Evaluation of sustained release property of organic acid-treated egg shell mixture (A) in simulated gastric cavity (1)>

The egg shell mixture (A) obtained in Example 7 was filled in a column having a pore diameter of 1 cm and a length of 15 cm as a simulated gastric cavity of an organism. Subsequently, 0.1 N aqueous HCl solution simulating a gastric juice as a solvent was passed through this column and separated by a low-pressure chromatography system (Bio-Rad Laboratories, Inc., BioLogic LP Chromatography System) over time. The flow rate of the solvent during the separation was 1.0 mL/min. Then, the calcium ion concentration In each separated solution was measured using Calcium C-test Wako (Wako Pure Chemical Industries, Ltd., Cat. No. 272-21801). The concentration of the organic add In each separated solution (lactic acid, acetic acid, and citric acid) was measured using F-Kit Lactic Acid, F-Kit Acetic Acid, and F-Kit Citric Acid (Roche Diagnostics K.K., Cat. Nos. 1112821, 148261, and 139076).

As a control, crushed egg shell particles untreated with organic acid (average particle size, 2 to 3 mm), which are the calcium-containing material used in Example 4, was similarly subjected to chromatography instead of the egg shell mixture (A) of Example 7. The amount of an eluted solvent (mL) and changes In the amount of calcium ions (integrated value) with elution of 0.1 N HCl were measured, and the amount of an eluted solvent (mL) and changes in the amount of each organic acid (integrated value) with elulion of 0.1 N HCl were measured. The measurement results regarding the amount of calcium ions (integrated value) are shown in Fig. 7. The measurement results regarding the amount of organic acids (integrated value) are shown in Fig. 8.

As shown in Fig. 7, it is found that the egg shell mixture (A) obtained in Example 7 provided more calcium ions in the column as compared with the crushed egg shell particles (control) from the stage of the start of measurement. Furthermore, it is found that the mixture (A) still provided more calcium ions than the control at 80 minutes after the start (amount of elution, 80 mL), which simulated retention in the gastric cavity. Furthermore, with a focus on the change in the amount of calcium ions (integrated value) over a period from the start of elution (amount of elution, 0 mL; elution time, 0 minute) to the completion of measurement (amount of elution, 80 mL; elution time, 80 minutes), it is found that the mixture (A) provides calcium ions in the column in a sustained-release manner without showing a rapid increase.

Meanwhile, as shown in Fig. 8, the changes in the amount of organic acids (integrated value) demonstrate that calcium lactate and calcium acetate from the egg shell mixture (A) obtained in Example 7 were dissolved first at the stage of the start of measurement. On the other hand, it is found that dissolution of calcium citrate was started gradually after the start of elution, increasing the integrated value of the concentration of the dissolved calcium citrate dissolved. Based on this result along with the results in Fig. 7, the sustained-release effect of calcium ions of the egg shell mixture (7) obtained in Example 7 can be attributed to the difference in elution time between organic acid calcium. That is, it is found that calcium ions are provided in the simulated gastric cavity over a long period of time due to the difference in solubility between the organic acid calcium, resulting in enhancement of the environment where a calcium content can be absorbed in the simulated gastric cavity.

### <Example 9: Evaluation of sustained release property of organic acid-treated egg shell mixture (A) in simulated gastric cavity (2)>

Sustained release property was evaluated in the same manner as in Example 8 using the egg shell mixture (A) obtained in Example 7 and crushed egg shell particles untreated with an organic acid (control), except that 0.05 N aqueous HCl solution was used as a solvent (simulated gastric juice) to simulate a state in which the gastric juice was diluted with drinking water in an organism.

The amount of an eluted solvent (mL) and changes in the amount of calcium ions (integrated value) with elution of 0.05 N HCl were measured, and the amount of an eluted solvent (mL) and changes in the amount of each organic acid (integrated value) with elution of 0.05 N HCl were measured. Among the obtained results, the measurement results regarding the amount of calcium ions (integrated value) are shown in Fig. 9. The measurement results regarding the amount of organic acids (integrated value) are shown in Fig. 10.

As shown in Fig. 9, in the system simulating that gastric juice was diluted with drinking water, the egg shell mixture (A) obtained in Example 7 eluted more calcium ions from the column from the stage of the start of measurement as compared with the crushed egg shell particles untreated with an organic acid (control). Furthermore, the changes over a period from the start of elution (amount of elution, 0 mL; elution time, 0 minute) to the completion of measurement (amount of elution, 80 mL; elution time, 80 minutes) demonstrated that the mixture (A) eluted calcium ions in the column in a sustained-release manner without showing a rapid increase. In the evaluation of sustained release property of the egg shell mixture (A) as compared with the crushed egg shell particles untreated with an organic add (control), comparison of the results of Examples 9 and 8 shows that the mixture of Example 9 provided a markedly high concentration of calcium ions to the simulated gastric cavity in a sustained-release manner as compared with the control.

On the other hand, as shown in Fig. 10, it was observed that calcium lactate and calcium acetate from the egg shell mixture (A) obtained in Example 7 were also dissolved first in the system using 0.05 N HCl of Example 9 at the stage of the start of measurement, and then calcium citrate was gradually dissolved. It is considered that this was associated with the sustained release property of calcium ions in the results of Fig. 9. In particular, in the system using 0.05 N HCl in Fig. 10, it was found that calcium citrate contained in the egg shell mixture (A) was dissolved in virtually the same amount of organic acids in Example 7 even under the condition that hydrochloric acid in the solvent was diluted to half the concentration in Example 7. This suggests that calcium citrate, in particular, elutes calcium ions relatively stably in a sustained-release manner regardless of the variations in the surrounding acidic condition.

As described above, in Example 9, the egg shell mixture (A) obtained in Example 7 depended largely on dissolution of calcium lactate and calcium acetate, which had relatively high solubility in water or an aqueous solution, at the early stage of elution of the solvent. At the middle stage of elution of the solvent, calcium citrate and calcium carbonate are dissolved, and calcium carbonate with relatively low solubility is dissolved at the late stage of elution of the solvent. It is considered that calcium ions are provided in the stomach In a sustained-release manner. Furthermore, more calcium ions can be provided to the gastric cavity of, in particular, organisms such poultry which drink a relatively large amount of water in summer, for example, by feeding a feed containing the egg shell mixture (A) of Example 7, the calcium absorption enhancer of the present invention than feeding a feed containing a crushed egg shell (calcium carbonate), and are provided in a sustained-release manner.

### <Example 10: Evaluation of sustained release property of organic acid-treated egg shell mixture (A) in simulated intestines (3)>

Sustained release property was evaluated in the same manner as in Example 8 using the egg shell mixture (A) obtained in Example 7 and crushed egg shell particles untreated with an organic acid (control), except that distilled water (simulated intestine solution) was used as a solvent simulating a state in which gastric acid was neutralized when a drink or a food was transported from the stomach to the duodenum.

The amount of an eluted solvent (mL) and changes in the amount of calcium ions (integrated value) with elution of distilled water were measured, and the amount of an eluted solvent (mL) and changes in the amount of each organic acid (integrated value) with elution of distilled water were measured. Among the obtained results, the measurement results regarding the amount of calcium ions (integrated value) are shown in Fig. 11. The measurement results regarding the amount of organic acids (integrated value) are shown in Fig. 12.

As shown in Fig. 11, it was found that, even when distilled water was used as a solvent, the egg shell mixture (A) obtained in Example 7 provided more calcium ions in the column as compared with the crushed egg shell particles (control) from the stage of the start of measurement.

Meanwhile, as shown in Fig. 12, the changes in the amounts of organic adds (integrated value) demonstrated that calcium lactate and calcium acetate from the egg shell mixture (A) obtained in Example 7 were dissolved first at the stage of the start of measurement even when distilled water was used as a solvent. On the other hand, it was found that, after the start of dissolution, calcium citrate was gradually dissolved, increasing the integrated value of the dissolved concentration.

### <Example 11: Production of calcium carbonate particles treated with an organic acid (water-soluble calcium particles (7))>

In a sealed bag, an aqueous solution of 50 mmol of lactic acid was gradually added to 50 g of calcium carbonate particles having an average particle size of 2 to 3 mm as a calcium-containing material and mixed, and then the mixture was allowed to stand and reacted at room temperature for 5 hours. Subsequently, the mixture was left and dried at 60°C overnight to obtain 55 g of water-soluble calcium particles (7) containing calcium lactate (solubility in water at 25°C, 4.8%).

### <Example 12: Production of calcium carbonate particles treated with an organic acid (water-soluble calcium particles (8))>

53 g of water-soluble calcium particles (8) containing calcium acetate (solubility in water at 25°C, 40%) was obtained In the same manner as in Example 11, except that an aqueous solution of 50 mmol of acetic add was used instead of the aqueous lactic acid solution.

### <Example 13: Production of calcium carbonate particles treated with an organic acid (water-soluble calcium particles (9))>

60 g of water-soluble calcium particles (9) containing calcium citrate (solubility in water at 25°C, 0.045%) was obtained in the same manner as in Example 11, except that an aqueous solution of 50 mmol of citric acid was used instead of the aqueous lactic acid solution.

### <Example 14: Production of calcium absorption enhancer (organic acid-treated calcium carbonate particle mixture (B))>

Equal amounts (0.5 g each) of the above-mentioned water-soluble calcium particles (7) obtained in Example 11. the above-mentioned water-soluble calcium particles (8) obtained in Example 12, the above-mentioned water-soluble calcium particles (9) obtained in Example 13, and calcium carbonate particles were collected and mixed uniformly to obtain a organic acid-treated calcium carbonate particle mixture (B).

The difference Y_{dif} in solubility between the water-soluble calcium particles in the calcium carbonate particle mixture treated with an organic acid (B) was 39.955.

### <Examples 15: Evaluation of sustained release property of calcium carbonate particle mixture treated with an organic acid (B) in simulated gastric cavity (4)>

Sustained release property was evaluated in the same manner as in Example 8, except that the calcium carbonate particle mixture (B) obtained in Example 14 was used as a calcium absorption enhancer instead of the egg shell mixture (A) obtained in Example 7. Using 0.1 N aqueous HCl solution as a solvent, the amount of solvent (mL) eluted in the simulated gastric cavity and changes in the amount of calcium ions (integrated value) with elution of the solvent were evaluated. The obtained results are shown in Fig. 13 together with the results of the egg shell mixture (A) of Example 7 obtained in above Example 8 and crushed egg shell particles untreated with an organic add (control).

As shown in Fig. 13, it was observed that the calcium carbonate particle mixture (B) of Example 14 eluted calcium ions in a sustained-release manner superior to that of the control as in the evaluation of sustained release property of the egg shell mixture (A) of Example 7.

### <Example 16: Evaluation of sustained release property of calcium carbonate particle mixture treated with an organic acid (B) in simulated gastric cavity (5)>

As a calcium absorption enhancer, the calcium carbonate particle mixture (B) obtained in Example 14 was used instead of the egg shell mixture (A) obtained in Example 7. Furthermore, as a solvent, 0.05 N aqueous HCl solution was used instead of 0.1 N aqueous HCl solution to simulate a state in which gastric juice in an organism was diluted with drinking water. Except for this condition, the amount of solvent (mL) eluted In the simulated gastric cavity and changes in the amount of calcium ions (integrated value) with elution of the solvent were evaluated In the same manner as in Example 8.

The obtained results are shown in Fig. 14 together with the results of the egg shell mixture (A) of Example 7 obtained in the above Example 9 and crushed egg shell particles untreated with an organic acid (control).

As shown in Fig. 14, it was observed that the calcium carbonate particle mixture (B) of Example 14 eluted calcium ions in a sustained-release manner superior to that of the control even under a relatively weaker acidic condition where the acidic concentration was diluted to half that in the conditions in Example 15, as In the evaluation of sustained release property of the egg shell mixture (A) of Example 7.

Furthermore, comparison of the results of the egg shell mixture (A) and the calcium carbonate mixture (B) in Figs. 13 and 14 shows that the use of crushed egg shell particles rather than calcium carbonate particles as a calcium-containing material resulted in superior sustained release property in the simulated gastric cavity under an acidic condition with a high concentration (0.1 N aqueous HCl solution) or a low concentration (0.05 N aqueous HCI solution). Furthermore, the difference in a sustained-release effect is more marked under relatively weak acidic conditions simulating an organism such as poultry after drinking a large amount of water. Therefore, the calcium absorption enhancer of the present invention can provide a more appropriate amount of calcium ions, not transiently, but in a sustained-release manner, for example, in the stomach of poultry or the like in which gastric juice is diluted with a large amount of drinking water in summer.

### <Example 17: Evaluation of sustained release property of calcium carbonate particle mixture treated with an organic acid (B) in simulated intestine (6)>

As a calcium absorption enhancer, the calcium carbonate particle mixture (B) obtained in Example 14 was used instead of the egg shell mixture (A) obtained in Example 7. Furthermore, as a solvent, distilled water was used instead of 0.1 N aqueous HCl solution to simulate a state in which gastric acid was neutralized when a drink or a food was transported from the stomach to the duodenum. Except for this condition, the amount of solvent (mL) eluted in the simulated gastric cavity and changes in the amount of calcium ions (integrated value) with elution of the solvent were evaluated in the same manner as in Example 8.

The obtained results are shown in Fig. 15 together with the above-mentioned results obtained in Example 10 of the egg shell mixture (A) of Example 7 and the crushed egg shell particles untreated with an organic acid (control).

As shown in Fig. 15, it was observed that the calcium carbonate particle mixture (B) of Example 14 eluted calcium ions in a sustained-release manner superior to that of the control even under the condition of distilled water (neutral) as in the evaluation of sustained release property of the egg shell mixture (A) of Example 7.

### <Examples 18: Evaluation of sustained release property of egg shell particles treated with an organic acid (water-soluble calcium particles (4)) in simulated gastric cavity (7)>

Sustained release property was evaluated in the same manner as in Example 8, except that the water-soluble calcium particles (4) obtained in Example 4 by treating with latic acid were used as a calcium absorption enhancer instead of the egg shell mixture (A) obtained in Example 7. Using 0.1 N aqueous HCl solution as a solvent, the amount of solvent (mL) eluted In the simulated gastric cavity and changes in the amount of calcium ions (integrated value) with elution of the solvent were evaluated. The obtained results are shown in Fig. 16 together with the results of the crushed egg shell particles untreated with an organic acid (control).

As shown in Fig. 16, It was observed that the lactic acid-treated water soluble calcium particles (4) of Example 4 eluted calcium ions in a sustained-release manner superior to that of the control as in the evaluation of sustained release property of the egg shell mixture (A) of Example 7.

### <Example 19: Evaluation of sustained release property of egg shell particles treated with an organic acid (water-soluble calcium particles (4)) in simulated gastric cavity (8)>

Sustained release property was evaluated in the same manner as in Example 9, except that the water-soluble calcium particles (4) obtained in Example 4 by treating with lactic acid were used as a calcium absorption enhancer instead of the egg shell mixture (A) obtained In Example 7. Using 0.05 N aqueous HCl solution as a solvent, the amount of solvent (mL) eluted in the simulated gastric cavity and changes in the amount of calcium ions (integrated value) with elution of the solvent were evaluated. The obtained results were shown in Fig. 17 together with the results of the crushed egg shell particles untreated with an organic acid (control).

As shown in Fig.17, it was observed that the lactic acid-treated water-soluble calcium particles (4) of Example 4 eluted calcium ions in a sustained-release manner superior to that of the control even under a relatively weaker acidic condition where the acidic concentration was diluted to half that in the conditions in Example 18 as in the evaluation of sustained release property of the egg shell mixture (A) of Example 7.

### <Examples 20: Evaluation of sustained release property of egg shell particles treated with an organic acid (water-soluble calcium particles (4)) in simulated intestine (9)>

Sustained release property was evaluated In the same manner as in Example 10, except that the water-soluble calcium particles (4) obtained in Example 4 by treating with lactic acid were used as a calcium absorption enhancer instead of the egg shell mixture (A) obtained in Example 7. Using distilled water as a solvent, the amount of solvent (mL) eluted in the simulated gastric cavity and changes in the amount of calcium ions (integrated value) with elution of the solvent were evaluated. The obtained results are shown in Fig. 18 together with the results of the crushed egg shell particles untreated with an organic acid (control).

As shown in Fig. 18, it was observed that the lactic acid-treated water-soluble calcium particles (4) of Example 4 eluted calcium ions in a sustained-release manner superior to that of the control even under the condition of distilled water (neutral) as in the evaluation of sustained release property of the egg shell mixture (A) of Example 7.

### Example 21: Production of egg shell particles treated with an organic acid (water-soluble calcium particles (10))>

As a calcium-containing material, an egg shell having an average particle size of 2 to 3 mm of a chicken (Leghorn) obtained by crushing in advance in a sealed bag using a blender (Zojirushi Corporation, model BM-FX08) and then passing through a sieve was used. As organic acids, a mixture solution of 15 g of 90% w/w lactic acid solution, 15 g of 10% w/w acetic acid solution, and 5 g of 3% w/w citric acid solution was used. The above-mentioned organic add mixture solution (35% w/w to the egg shell) was gradually added to 100 g of the above-mentioned egg shell and mixed, and then the mixture was allowed to stand and reacted at room temperature for 5 hours. Subsequently, the mixture was left and dried at 60°C overnight to obtain 120 g of water-soluble calcium particles (10) containing calcium lactate (solubility in water at 25°C, 4.8%), calcium acetate (solubility in water at 25°C, 40%), and calcium citrate (solubility in water at 25°C, 0.045%).

### <Example 22: Evaluation of sustained release property of egg shell particles treated with an organic acid (water-soluble calcium particles (10)) in simulated gastric cavity (10)>

Sustained release property was evaluated in the same manner as in Example 8, except that the water-soluble calcium particles (10) obtained in Example 21 were used as a calcium absorption enhancer instead of the egg shell mixture (A) obtained in Example 7. Using 0.1 N aqueous HCl solution as a solvent, the amount of solvent (mL) eluted in the simulated gastric cavity and changes in the amount of calcium ions (integrated value) with elution of the solvent were evaluated. The obtained results are shown in Fig. 19 together with the results, of the crushed egg shell particles untreated with an organic acid (control).

As shown in Fig. 19, it was observed that the water-soluble calcium particles (10) of Example 21 eluted calcium ions in a sustained-release manner superior to that of the control in the same manner as in the evaluation of sustained release property of the egg shell mixture (A) of Example 7.

### <Example 23: Evaluation of sustained release property of egg shell particles treated with an organic acid (water-soluble calcium particles (10)) in simulated gastric cavity (11)>

Sustained release property was evaluated in the same manner as in Example 9, except that the water-soluble calcium particles (10) obtained in Example 21 were used as a calcium absorption enhancer instead of the egg shell mixture (A) obtained in Example 7. Using 0.05 N aqueous HCl solution as a solvent, the amount of solvent (mL) eluted in the simulated gastric cavity and changes in the amount of calcium ions (integrated value) with elution of the solvent were evaluated. The obtained results are shown in Fig. 20 together with the results of the crushed egg shell particles untreated with an organic acid (control).

As shown in Fig. 20, it was observed that the organic acid mixture-treated water-soluble calcium particles (10) of Example 21 eluted calcium ions in a sustained-release manner superior to that of the control even under a relatively weaker acidic condition where the acidic concentration was diluted to half that in the conditions in Example 22 as in the evaluation of sustained release property of the egg shell mixture (A) of Example 7.

### <Example 24: Evaluation of sustained release property of egg shell particles treated with an organic acid (water-soluble calcium particles (10)) in simulated intestine (12)>

Sustained release property was evaluated in the same manner as in Example 10, except that the water-soluble calcium particles (10) obtained in Example 21 were used as a calcium absorption enhancer instead of the egg shell mixture (A) obtained in Example 7. Using distilled water as a solvent, the amount of solvent (mL) eluted in the simulated intestine and changes in the amount of calcium ions (integrated value) with elution of the solvent were evaluated. The obtained results are shown in Fig. 21 together with the results of the crushed egg shell particles untreated with an organic acid (control).

As shown in Fig. 21, it was observed that the organic acid mixture-treated water-soluble calcium particles (10) of Example 21 eluted calcium ions in a sustained-release manner superior to that of the control even under the condition of distilled water (neutral) as in the evaluation of sustained release property of the egg shell mixture (A) of Example 7.

### <Example 25: Production of whole-grain cookie>

A whole-grain cookies were made as follows using the water-soluble calcium particles (1) obtained in Example 1 and materials shown in Table 1.

**Table 1**

| Material | Amount |
|---|---|
| Materials to be combined (a) | |
| · Whole grain | 80 g |
| · Wheat bran | 10 g |
| · Water-soluble calcium particles (1) of Example 1 | 10 g |
| · Baking powder | Tea spoon 1/3 |
| · Cinnamon | Tea spoon 1/3 |
| Unsalted butter | 40 g |
| Sugar | 40 g |
| Whole egg | 1/2 |
| Egg white | 1/2 (for icing) |

First, unsalted butter was placed in a bowl and beaten into a creamy consistency, and sugar was added thereto and mixed while rubbing in. Then, the materials to be combined (a) shown in Table 1, which had been sifted in advance, were added and mixed lightly. Then, the obtained dough was placed in a polyethylene bag and stored in a refrigerator for 30 minutes. Subsequently, the dough was removed from the refrigerator and then from the bag, roiled out with a rolling pin to a thickness of 3 mm, and cut into pieces.

The cut dough pieces were placed on a baking sheet, and the surface was coated with beaten egg white. After the surface was continued to be dry, egg white was applied again. Then, the dough was baked in an oven set at 170°C for 12 minutes to obtain a whole-grain cookies containing the water-soluble calcium particles (1) of Example 1.

### <Example 26: Production of no bake cheesecake>

A no bake cheesecake was made as follows using the water-soluble calcium particles (1) obtained in Example 1 and materials shown in Table 2.

**Table 2**

| Material | Amount |
|---|---|
| <Cheese filling> | |
| · Cream cheese | 250 g |
| · Fresh cream | 200 cc |
| · Plain yogurt | 200 g |
| · Sugar | 80 g |
| · Lemon juice | 30 cc |
| · Gelatin powder | 7 g |
| · Vanilla flavoring | As required |

| <Cookie dough> | |
|---|---|
| · Commercially available cookie | 90 g |
| · Unsalted butter | 50 g |
| · Water-soluble calcium particles (1) of Example 1 | 10 g |

Commercially available cookies were placed in a polyethylene bag and crushed and mixed with the water-soluble calcium particles (1) obtained in Example 1 and butter to prepare a cookie dough. The cookie dough was spread on the bottom of a cake tin having a diameter of 18 cm.

Cream cheese covered with a plastic wrap was heated in a microwave oven until softened, sugar was added thereto, and the mixture was beaten well with a beater until becoming smooth overall. Then, vanilla flavoring, lemon juice, fresh cream, and plain yogurt were added thereto and further mixed to obtain filling. Powder gelatin dissolved in advance was added to the filling, and all the ingredients were thoroughly mixed to obtain cheese filling.

The cheese filling was poured into the cake tin, in which the above-mentioned cookie dough was spread, and stored in a refrigerator until set to obtain a target no bake cheesecake containing the water-soluble calcium particles (1) of Example 1.

### <Example 27: Production of mixed feed composition>

A mixed feed composition for breeding adult chickens having the following composition was prepared using the water-soluble calcium particles (1) obtained in Example 1.

**Table 3**

| Material | Amount |
|---|---|
| Crop (mixture of maize and Milo) | 61 % by mass |
| Vegetable oil meal (mixture of soybean oil meal and corn gluten meal) | 24 % by mass |
| Animal feed (fish meal) | 4 % by mass |
| Bran (rice bran) | 0.2 % by mass |
| Other ingredients (mixture of the following ingredients) Calcium carbonate, animal oils and fats, calcium phosphate, common salt, paprika extract, anhydrous silicic acid, vitamins, minerals, amino acids, enzyme formulation, anti-oxidizing agent (ethoxyquin) | 11.8 % by mass |

A mixed feed composition for breeding adult chickens was obtained by mixing the ingredients shown in Table 3 uniformity.

### Industrial Applicability

The calcium absorption enhancer of the present invention can be used for the purpose of calcium fortification in humans or domestic animals. In particular, the calcium absorption enhancer of the present invention is useful in the technical field of foods and feeds.

## Claims

1. A calcium absorption enhancer, **characterized by** comprising as an active ingredient water-soluble calcium particles that can release calcium ions in an aqueous solution in a sustained-release manner.

2. The calcium absorption enhancer according to claim 1, wherein the surface of the water-soluble calcium particles has a microscopic void structure.

3. The calcium absorption enhancer according to claim 2, wherein the microscopic void structure has at least one structure selected from the group consisting of a capillary structure, a granular structure, and a spongy structure.

4. The calcium absorption enhancer according to claim 2 or 3, wherein the water-soluble calcium particles are obtained by treating a calcium-containing material with an add.

5. The calcium absorption enhancer according to claim 4, wherein the calcium-containing material is a biological material.

6. The calcium absorption enhancer according to claim 5, wherein the biological material is at least one material selected from the group consisting of an egg shell, a seashell, an echinoderm shell, and an echinoderm spine.

7. The calcium absorption enhancer according to any one of claims 4 to 6, wherein the acid is an organic acid.

8. The calcium absorption enhancer according to claim 7, wherein the organic acid is at least one acid selected from the group consisting of citric acid, acetic acid, lactic acid, formic acid, malic acid, propionic acid, butyric add, gluconic acid, succinic acid, and valeric acid.

9. The calcium absorption enhancer according to any one of claims 1 to 8, wherein the water-soluble calcium particles have an average particle size of 0.1 to 10 mm.

10. The calcium absorption enhancer according to any one of claims 1 to 9, wherein the calcium absorption enhancer is used for administration to a domestic animal.

11. The calcium absorption enhancer according to any one of claims 1 to 9, wherein the calcium absorption enhancer is used for administration to a human.

12. The calcium absorption enhancer according to claim 1, wherein the water-soluble calcium particles are mixture of two or more water-soluble calcium compounds having different solubility In water or an aqueous solution.

13. The calcium absorption enhancer according to claim 12, wherein the water-soluble calcium compounds are an organic acid calcium.

14. The calcium absorption enhancer according to claim 13, wherein the organic acid calcium is at least one selected from the group consisting of calcium citrate, calcium acetate, calcium lactate, calcium formate, calcium malate, calcium propionate, calcium butyrate, calcium gluconate, calcium succinate, and calcium valerate.

15. The calcium absorption enhancer according to claim 12, wherein the water-soluble calcium particles have a microscopic void structure in their surface.

16. The calcium absorption enhancer according to claim 15, wherein the microscopic void structure has at least one structure selected from the group consisting of a capillary structure, a granular structure, and a spongy structure.

17. The calcium absorption enhancer according to claim 15 or 16, wherein the water-soluble calcium particles are obtained by treating a calcium-containing material with an acid.

18. The calcium absorption enhancer according to claim 17, wherein the calcium-containing material is a biological material.

19. The calcium absorption enhancer according to claim 18, wherein the biological material is at least one material selected from the group consisting of an egg shell, a seashell, an echinoderm shell, and an echinoderm spine.

20. The calcium absorption enhancer according to any one of claims 17 to 19, wherein the acid is an organic acid.

21. The calcium absorption enhancer according to claim 20, wherein the organic acid is at least one selected from the group consisting of nitric add, acetic acid, lactic acid, formic acid, malic acid, propionic acid, butyric acid, gluconic acid, succinic acid, and valeric add.

22. The calcium absorption enhancer according to any one or claims 12 to 21, wherein the water-soluble calcium particles have an average particle size of 0.1 to 10 mm.

23. The calcium absorption enhancer according to any one of claims 12 to 22, wherein the calcium absorption enhancer is used for administration to a domestic animal.

24. The calcium absorption enhancer according to any one of claims 12 to 22, wherein the calcium absorption enhancer is used for administration to a human.
